(19) Europäisches Patentamt
European Patent Office
Office européen des brevets

(11) EP 3 118 161 B1

(12) **EUROPEAN PATENT SPECIFICATION**

(45) Date of publication and mention of the grant of the patent:
**16.05.2018 Bulletin 2018/20**

(51) Int Cl.:
***C01F 11/18*** (2006.01) ***C09C 1/02*** (2006.01)
***D21H 17/67*** (2006.01) ***D21H 19/38*** (2006.01)

(21) Application number: **15177344.7**

(22) Date of filing: **17.07.2015**

(54) **HIGH SOLIDS PCC WITH DEPOLYMERIZED CARBOXYLATED CELLULOSE**

PCC MIT HOHEM FESTSTOFFGEHALT MIT DEPOLYMERISIERTER CARBOXYLIERTER CELLULOSE

PCC À HAUTE TENEUR EN SOLIDES À BASE DE CELLULOSE CARBOXYLÉE DÉPOLYMÉRISÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(43) Date of publication of application:
**18.01.2017 Bulletin 2017/03**

(73) Proprietor: **Omya International AG**
**4665 Oftringen (CH)**

(72) Inventors:
- **MAURER, Marc**
  **68128 Village-Neuf (FR)**
- **SCHLOTTERBACH, Thomas**
  **9500 Villach (AT)**
- **JACQUEMET, Christian**
  **69005 Lyon (FR)**

(74) Representative: **Müller-Dyck, Martina**
**Maiwald Patentanwalts GmbH**
**Elisenhof**
**Elisenstraße 3**
**80335 München (DE)**

(56) References cited:
**WO-A1-2007/067146** **WO-A1-2015/062978**
**US-A1- 2014 242 387**

- **DATABASE CA [Online] CHEMICAL ABSTRACTS SERVICE, COLUMBUS, OHIO, US; 2012, TAN, JIAN-YUN ET AL: "Preparation and charaterisation of spherical cellulose sodium", XP002752677, retrieved from STN Database accession no. 157:233785 & TAN, JIAN-YUN ET AL: "Preparation and charaterisation of spherical cellulose sodium", ZHEJIANG DAXUE XUEBAO, GONGXUEBAN , 46(2), 226-231 CODEN: ZDXGFS; ISSN: 1008-973X, 2012, DOI: 10.3785/J.ISSN.1008-973X.2012.02.007 10.3785/J.ISSN.1008-973X.2012.02.007**

EP 3 118 161 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to a process for producing an aqueous suspension of precipitated calcium carbonate.

Calcium carbonate is one of the most commonly used additives in the paper, paint and plastics industries. While naturally occurring ground calcium carbonate (GCC) is usually used as a filler in many applications, synthetically manufactured precipitated calcium carbonate (PCC) may be tailor-made with respect to its morphology and particle size allowing this materials to fulfil additional functions.

Commonly known PCC production processes including the steps of slaking quicklime with water, and subsequently precipitating calcium carbonate by passing carbon dioxide through the resulting calcium hydroxide suspension, produce only PCC slurries with low solids content. Therefore, these processes typically comprise a subsequent solids concentration step in order to obtain a more concentrated PCC slurry, for example, for shipping the PCC slurry. However, such additional solids concentration steps are energy-consuming and cost-intensive and require equipment such as a centrifuge, which is expensive and needs high maintenance. Furthermore, mechanical dewatering processes using centrifuges can destroy the structure of the formed PCC, for example, in case of clustered scalenohedral PCC.

WO 2011/121065 A1 discloses a process for preparing PCC comprising inter alia the step of preparing an aqueous suspension of PCC seeds by carbonating a suspension of calcium hydroxide in the presence of strontium hydroxide. A process for producing PCC, wherein the addition rate of the calcium carbonate slurry to the reaction vessel is such that a certain electrical conductivity is maintained in the reaction vessel, is described in EP 2 537 900 A1.

US 2011/158890 A1 describes a method to manufacture PCC involving the use of a comb polymer, which reduces the carbonation time of the PCC. A grinding agent for grinding coarse lime is disclosed in EP 0 313 483 A1. EP 2 447 213 A1 relates to the production of high purity PCC involving the step of slaking lime with an aqueous ammonium chloride solution.

WO 2013/142473 A1 relates to a process comprising the steps of slaking quick lime to obtain slaked lime, and subjecting the slaked lime, without agitation, without prior cooling in a heat exchanger, and in the absence of any additives, to carbonation with carbon dioxide gas to produce PCC. PCC production processes including additives are disclosed in US patents no. 6294143, 5232678, and 5558850. A method for producing slaked lime by slaking lime with a polymer having anionic groups is described in JP 2008/074629 A. EP 0 844 213 A1 discloses a method of producing a precipitate of an alkaline earth metal compound involving the use of a dispersing agent.

WO 2010/018432 A1 discloses a process to prepare precipitated calcium carbonate implementing low charge acrylate and/or maleinate-containing polymers. A process for producing platy precipitated calcium carbonate involving the step of adding a polyacrylate to a suspension of calcium hydroxide prior to the completion of carbonation is described in WO 2005/000742 A1. WO 2004/106236 A1 relates to a process for producing platy precipitated calcium carbonate involving the step of adding a dry condensed phosphate additive to a suspension of calcium hydroxide prior to the completion of carbonation.

It is further known from EP 2 929 980 A1 that an aqueous suspension of precipitated calcium carbonate can be prepared by carbonating a milk of lime, which has been obtained by mixing water, a calcium oxide containing material, at least one water-soluble polymer having a molecular weight $M_w$ in the range from 200 to 6 500 g/mol, and at least one slaking additive, wherein the calcium oxide containing material and the water are mixed in a weight ratio from 1:2.5 to 1:6. The at least one water-soluble polymer has the chemical structure of formula (I)

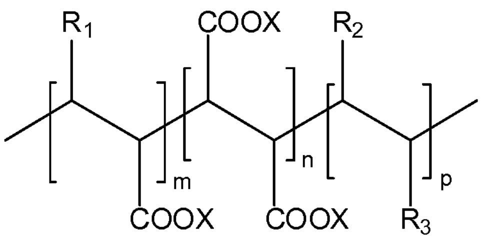 (I),

wherein n, m, and p are integers and at least one of n, m, or p is greater than zero and n+m+p is less than or equal to 70,

$R_1$ is H or $CH_3$,

$R_2$ is H or $CH_3$;

$R_3$ is -C(=O)-O-$R_4$ or -C(=O)-NH-$R_4$, wherein $R_4$ is a $C_1$ to $C_{20}$ alkyl group, a $C_3$ to $C_{20}$ cycloalkyl group and/or a $C_6$ to $C_{30}$ aryl group, being optionally substituted with one or more sulfonate groups, and wherein the cycloalkyl group and/or the aryl group comprises one ring or several rings, which are linked to each other, and

X is H and/or M, wherein M is Na, K, Li, Mg, and/or Ca, and

wherein the structural units

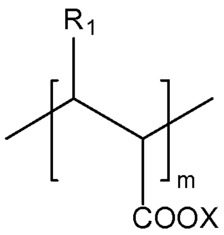 , 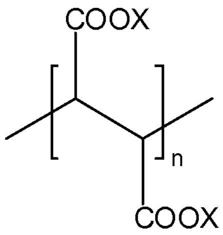 , 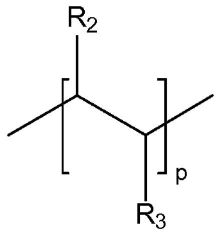

are arranged randomly, regularly and/or in blocks.

Furthermore, reference is made to EP 3 061 729 A1, which describes a process for producing an aqueous suspension of precipitated calcium carbonate, wherein a milk of lime is carbonated, which is obtained from mixing water, a calcium oxide containing material, and at least one cationic polymer.

A method for preparing calcium carbonate using additives and precipitation agents is also known from KR100958593 B1. The method comprises an elution step of adding, into a container equipped with an agitator, a lime-based byproduct comprising steel-making slag or quick lime (CaO) dust, water at a ratio of 20 to 50 1 per 60 to 100 g of the lime-based byproduct, at least one additive selected from the group consisting of sodium trimetaphosphate, sodium hexametaphosphate, sodium polycarbonate, ammonium polycarbonate, sodium polycarboxylate, formic acid, succinic acid, sucrose fatty acid ester, sodium citrate, ammonium citrate, and ammonium chloride in an amount of 0.01 to 10.0 parts by weight with respect to 100 parts by weight of the lime-based byproduct, and at least one precipitating agent selected from the group consisting of a cationic precipitating agent, an anionic precipitating agent, and a nonionic precipitating agent in an amount of 0.01 to 3.0 parts by weight with respect to 100 parts by weight of the lime-based byproduct and performing a mixing, to elute calcium ions; a precipitation step of standing the resulting mixture for a predetermined period of time after the completion of mixing the lime-based byproduct, water, the additive, and the precipitating agent in the elution step to precipitate the lime-based byproduct; a carbonation step of separating a clear supernatant eluate after the completion of the precipitation step and then feeding carbon dioxide into the eluate to cause a reaction until the eluate is at pH 9; and a calcium carbonate collection step of collecting calcium carbonate precipitated on the bottom after the completion of the carbonation step.

However, said methods have the drawback that an additive in combination with a precipitating agent has to be added to the calcium oxide comprising material. Furthermore, especially in KR100958593 B1, it is described that the obtained mixture is separated in a precipitated bottom portion and a clear supernatant eluate. The carbonating and the resulting precipitation of calcium carbonate is then only carried out on the obtained clear supernatant eluate in order to obtain a calcium carbonate product comprising less impurities. Thus, said method requires additional separation steps allowing a separation of solid and liquid phase during processing which results in a more time and cost-consuming production of precipitated calcium carbonate. Furthermore, it is to be noted that the precipitation agent is used in said method for adsorbing the slurry suspended in water to coagulate and precipitate the slurry through cross-linking, which enables a rapid solid-liquid separation. However, due to the following separation of the liquid and solid phases and carbonating of only the liquid phase, i.e. the clear supernatant eluate, the precipitation agent is not present in the carbonating step and is thus not used for the following precipitation of calcium carbonate.

WO 2007/067146 A1 is concerned with a method of producing precipitated calcium carbonate, wherein the carbonation of calcium hydroxide is performed in the presence of starch or carboxymethylcellulose.

In view of the foregoing, there is a continuous need for processes providing precipitated calcium carbonate, and especially those which allow the direct production of PCC suspensions with a high solids content without an additional separation or concentration step.

Accordingly, it is an object of the present invention to provide a process for producing a PCC suspension with a high solids content at an acceptable viscosity. It is also desirable that said process does not require any mechanical or thermal concentration step during processing. It is also desirable that said process does not require any separation step during processing, especially before the milk of lime is carbonated. It is also desirable that said process does not affect the kinetics of the carbonation step in a negative way and/or does not impair the crystallographic structure of the PCC.

The foregoing and other objects are solved by the subject-matter as defined herein in the independent claims.

According to one aspect of the present invention, a process for producing an aqueous suspension of precipitated calcium carbonate is provided, the process comprising the steps of:

i) providing a calcium oxide containing material,
ii) providing at least one depolymerized carboxylated cellulose having a molecular weight $M_w$ in the range from 10

000 to 40 000 g/mol,

iii) preparing a milk of lime by mixing water, the calcium oxide containing material of step i), and the at least one depolymerized carboxylated cellulose of step ii) to obtain a milk of lime, wherein the calcium oxide containing material and the water are mixed in a weight ratio from 1:1 to 1:12, and

iv) carbonating the milk of lime obtained in step iii) to form an aqueous suspension of precipitated calcium carbonate.

Advantages embodiment of the present invention are defined in the corresponding sub-claims.

According to one embodiment step iii) comprises the steps of: a1) mixing the at least one depolymerized carboxylated cellulose of step ii) with water, and a2) adding the calcium oxide containing material of step i) to the mixture of step a1), or b1) mixing the calcium oxide containing material of step i), and the at least one depolymerized carboxylated cellulose of step ii), and b2) adding water to the mixture of step b1), or c) mixing the calcium oxide containing material of step i), the at least one depolymerized carboxylated cellulose of step ii) and water simultaneously.

According to one embodiment the process further comprises step v) of adding at least one slaking additive to process step iii), preferably the at least one slaking additive is selected from the group consisting of organic acids, organic acid salts, sugar alcohols, monosaccharides, disaccharides, polysaccharides, gluconates, phosphonates, lignosulfonates, and mixtures thereof. According to another embodiment the milk of lime obtained in step iii) has a Brookfield viscosity from 1 to 1000 mPa·s at 25°C, more preferably from 5 and 800 mPa·s at 25°C, and most preferably from 10 and 500 mPa·s at 25°C, and/or the suspension of PCC obtained in step iv) has a Brookfield viscosity of less than or equal to 1600 mPa·s at 25°C, more preferably less than or equal to 1500 mPa·s at 25°C, and most preferably less than or equal to 1400 mPa·s at 25°C.

According to one embodiment the suspension of PCC obtained in step iv) has a solids content of at least 10 wt.-%, preferably from 15 to 70 wt.-%, more preferably from 19 to 60 wt.-%, even more preferably from 21 to 50 wt.-%, and most preferably from 24 to 42 wt.-%, based on the total amount of the suspension. According to another embodiment the depolymerized carboxylated cellulose has a polydispersity index from 2 to 10, preferably from 2 to 8, more preferably from 2.5 to 6, and most preferably from 3 to 5. According to still another embodiment the depolymerized carboxylated cellulose has degree of carboxylation from 0.2 to 2, preferably from 0.4 to 1.8, more preferably from 0.5 to 1.6, and most preferably from 0.6 to 1.4.

According to one embodiment the depolymerized carboxylated cellulose has molecular weight $M_w$ in the in the range from 13 000 to 35 000 g/mol, and preferably in the range from 13 000 to 25 000 g/mol. According to another embodiment the depolymerized carboxylated cellulose is provided in form of a solution having a solids content from 10 to 60 wt.-%, based on the total weight of the solution, preferably from 25 to 45 wt.-%, more preferably from 30 to 40 wt.-%, and most preferably from 31 to 35 wt.-%, and/or is added in an amount from 0.001 to 5 wt.-%, based on the total weight of the calcium oxide containing material in the milk of lime, preferably from 0.01 to 2 wt.-%, more preferably from 0.05 to 1 wt.-%, and most preferably from 0.1 to 0.5 wt.-%.

According to one embodiment the depolymerized carboxylated cellulose is prepared by depolymerizing a high molecular weight carboxylated cellulose in a process comprising the following steps: I) providing a high molecular weight carboxylated cellulose having a molecular weight of more than 40 000 g/mol and a degree of carboxylation in the range from 0.2 to 2, II) providing a peroxide selected from hydrogen peroxide and/or an alkali metal salt thereof, III) mixing the high molecular weight carboxylated cellulose of step I) and/or the peroxide of step II) and water incrementally and in any order at a reaction temperature from 50 to 85°C, IV) maintaining the temperature of the mixture obtained from step III) until complete consumption of the peroxide, V) cooling the mixture to a temperature of below 50°C, and VI) optionally, neutralizing the obtained depolymerized carboxylated cellulose.

According to one embodiment the depolymerized carboxylated cellulose is a carboxymethyl derivate and/or carboxymethyl hydroxypropyl derivate and/or carboxymethyl hydroxyethyl derivate of cellulose, preferably the depolymerized carboxylated cellulose is depolymerized carboxymethylcellulose. According to another embodiment the process further comprises step vi) of separating the precipitated calcium carbonate from the aqueous suspension obtained in step iv), and optionally step vii) of drying the separated precipitated calcium carbonate obtained in step vi).

According to one embodiment the process further comprises a step viii) of contacting at least a part of the surface of the obtained precipitated calcium carbonate with at least one hydrophobising agent after step iv) and/or after step vi), if present, and/or during and/or after step vii), if present, preferably the at least one hydrophobising agent is selected from the group consisting of an aliphatic carboxylic acid having a total amount of carbon atoms from $C_4$ to $C_{24}$ and/or reaction products thereof, a mono-substituted succinic anhydride consisting of succinic anhydride mono-substituted with a group selected from a linear, branched, aliphatic and cyclic group having a total amount of carbon atoms from at least $C_2$ to $C_{30}$ in the substituent and/or reaction products thereof, a phosphoric acid ester blend of one or more phosphoric acid mono-ester and/or reaction products thereof and one or more phosphoric acid di-ester and/or reaction products thereof, polyhydrogensiloxane and reaction products thereof, an inert silicone oil, preferably polydimethylsiloxane, and mixtures thereof.

It should be understood that for the purpose of the present invention, the following terms have the following

meaning:
A "calcium oxide containing material" in the meaning of the present invention can be a mineral or a synthetic material having a content of calcium oxide of at least 50 wt.-%, preferably 75 wt.-%, more preferably 90 wt.-%, and most preferably 95 wt.-%, based on the total weight of the calcium oxide containing material. For the purpose of the present invention, a "mineral material" is a solid substance having a definite inorganic chemical composition and characteristic crystalline and/or amorphous structure.

"Ground calcium carbonate" (GCC) in the meaning of the present invention is a calcium carbonate obtained from natural sources, such as limestone, marble, or chalk, and processed through a wet and/or dry treatment such as grinding, screening and/or fractionation, for example by a cyclone or classifier.

Throughout the present document, the "particle size" of precipitated calcium carbonate or other particulate materials is described by its distribution of particle sizes. The value $d_x$ represents the diameter relative to which x % by weight of the particles have diameters less than $d_x$. This means that the $d_{20}$ value is the particle size at which 20 wt.-% of all particles are smaller, and the $d_{98}$ value is the particle size at which 98 wt.-% of all particles are smaller. The $d_{98}$ value is also designated as "top cut". The $d_{50}$ value is thus the weight median particle size, i.e. 50 wt.-% of all grains are smaller than this particle size. For the purpose of the present invention the particle size is specified as weight median particle size $d_{50}$ unless indicated otherwise. For determining the weight median particle size $d_{50}$ value or the top cut particle size $d_{98}$ value a Sedigraph 5100 or 5120 device from the company Micromeritics, USA, can be used.

"Precipitated calcium carbonate" (PCC) in the meaning of the present invention is a synthesized material, generally obtained by precipitation following a reaction of carbon dioxide and calcium hydroxide (hydrated lime) in an aqueous environment or by precipitation of a calcium- and a carbonate source in water. Additionally, precipitated calcium carbonate can also be the product of introducing calcium and carbonate salts, calcium chloride and sodium carbonate for example, in an aqueous environment. PCC may have a vateritic, calcitic or aragonitic crystalline form. PCCs are described, for example, in EP 2 447 213 A1, EP 2 524 898 A1, EP 2 371 766 A1, or WO 2013/142473 A1.

A "suspension" or "slurry" in the meaning of the present invention comprises insoluble solids and water, and optionally further additives, and usually contains large amounts of solids and, thus, is more viscous and can be of higher density than the liquid from which it is formed.

For the purpose of the present invention, the "solids content" of a liquid composition such as a suspension or slurry is a measure of the amount of material remaining after all the solvent or water has been evaporated.

Throughout the present document, the "degree of carboxylation" is specified in respect to the total amount of hydroxyl groups per unmodified monomer unit of the original cellulose. A "degree of carboxylation" of 1 means that one of the three hydroxyl groups of the unmodified monomer unit of the original cellulose is carboxylated.

A "carboxylated cellulose" in the meaning of the present invention is a cellulose that has been chemically modified and includes carboxylic units, for example, carboxymethyl units ($-CH_2COOH$).

The term "depolymerized carboxylated cellulose" refers to a carboxylated cellulose, which is obtained by depolymerisation or degradation of a carboxylated cellulose having a molecular weight $M_w$ of more than 40 000 g/mol (measured by Gel Permeation, GPC). The term "depolymerized carboxymethylcellulose" (depolymerized CMC) refers to a carboxymethylcellulose (CMC), which is obtained by depolymerisation or degradation of a carboxymethylcellulose having a molecular weight $M_w$ of more than 40 000 g/mol (measured by Gel Permeation, GPC).

The term "polydispersity index" as used in the context of the present invention is a measure of the distribution of molecular weights in a given polymer sample, e.g. carboxylated cellulose sample. When the polydispersity index is one, the molecular weight distribution of all polymers in the sample is monodisperse, i.e. all polymers have the same chain length, and thus, molecular weight. However, for real polymers, the polydispersity index is commonly greater than one and represents the ratio of $M_w/M_n$ which is the weight average molecular weight divided by the number average molecular weight of the polymer.

A "specific BET surface area" (SSA) in the meaning of the present invention is defined as the surface area of the precipitated calcium carbonate particles divided by the mass of PCC particles. As used therein the specific surface area is measured by adsorption using the BET isotherm (ISO 9277:1995) and is specified in $m^2/g$.

For the purpose of the present invention, the term "viscosity" or "Brookfield viscosity" refers to Brookfield viscosity. The Brookfield viscosity is for this purpose measured by a Brookfield DV-II+ Pro viscometer at 25C $\pm$ 1°C at 100 rpm using an appropriate spindle of the Brookfield RV-spindle set and is specified in mPa·s. Based on his technical knowledge, the skilled person will select a spindle from the Brookfield RV-spindle set which is suitable for the viscosity range to be measured.

For example, for a viscosity range between 200 and 800 mPa·s the spindle number 3 may be used, for a viscosity range between 400 and 1 600 mPa·s the spindle number 4 may be used, for a viscosity range between 800 and 3 200 mPa·s the spindle number 5 may be used, for a viscosity range between 1 000 and 2 000 000 mPa·s the spindle number 6 may be used, and for a viscosity range between 4 000 and 8 000 000 mPa·s the spindle number 7 may be used.

Unless specified otherwise, the term "drying" refers to a process according to which at least a portion of water

is removed from a material to be dried such that a constant weight of the obtained "dried" material at 120 °C is reached. Moreover, a "dried" material may be further defined by its total moisture content which, unless specified otherwise, is less than or equal to 1.0 wt.-%, preferably less than or equal to 0.5 wt.-%, more preferably less than or equal to 0.2 wt.-%, and most preferably between 0.03 and 0.07 wt.-%, based on the total weight of the dried material.

The "total moisture content" of a material refers to the percentage of moisture (i.e. water) which may be desorbed from a sample upon heating to 220 °C.

In the meaning of the present invention, "stable in an aqueous suspension having a pH of 12 and a temperature of 95°C" means that the depolymerized carboxylated cellulose maintains its physical properties and chemical structure when added to an aqueous suspension having a pH of 12 and a temperature of 95°C. For example, the polymer maintains its dispersing qualities and is not degraded under said conditions.

Where the term "comprising" is used in the present description and claims, it does not exclude other non-specified elements of major or minor functional importance. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Whenever the terms "including" or "having" are used, these terms are meant to be equivalent to "comprising" as defined above.

Where an indefinite or definite article is used when referring to a singular noun, e.g. "a", "an" or "the", this includes a plural of that noun unless something else is specifically stated.

Terms like "obtainable" or "definable" and "obtained" or "defined" are used interchangeably. This e.g. means that, unless the context clearly dictates otherwise, the term "obtained" does not mean to indicate that e.g. an embodiment must be obtained by e.g. the sequence of steps following the term "obtained" even though such a limited understanding is always included by the terms "obtained" or "defined" as a preferred embodiment.

The inventive process for producing an aqueous suspension of precipitated calcium carbonate comprises the steps of i) providing a calcium oxide containing material, ii) providing at least one depolymerized carboxylated cellulose having a molecular weight $M_w$ in the range from 10 000 to 40 000 g/mol, iii) preparing a milk of lime by mixing water, the calcium oxide containing material of step i), and the at least one depolymerized carboxylated cellulose of step ii) to obtain a milk of lime, wherein the calcium oxide containing material and the water are mixed in a weight ratio from 1:1 to 1:12, and iv) carbonating the milk of lime obtained in step iii) to form an aqueous suspension of precipitated calcium carbonate.

In the following details and preferred embodiments of the inventive process will be set out in more details.

<u>Process step i): provision of a calcium oxide containing material</u>

According to step i) of the process of the present invention, a calcium oxide containing material is provided.

The calcium oxide containing material of step i) can be obtained by calcining a calcium carbonate containing material. Calcination is a thermal treatment process applied to calcium carbonate containing materials in order to bring about a thermal decomposition resulting in the formation of calcium oxide and gaseous carbon dioxide. Calcium carbonate containing materials which may be used in such a calcinations process are those selected from the group comprising precipitated calcium carbonates, natural calcium carbonate containing minerals such as marble, limestone and chalk, and mixed alkaline earth carbonate minerals comprising calcium carbonate such as dolomite, or calcium carbonate rich fractions from other sources. It is also possible to subject a calcium carbonate containing waste material to a calcinations process in order to obtain a calcium oxide containing material. Calcium carbonate decomposes at about 1 000 °C to calcium oxide (commonly known as quicklime). The calcination step may be carried out under conditions and using equipment well-known to the person skilled in the art. Generally, calcination may be carried out in furnaces or reactors (sometimes referred to as kilns) of various designs including shaft furnaces, rotary kilns, multiple hearth furnaces, and fluidized bed reactors.

The end of the calcination reaction may be determined, e.g. by monitoring the density change, the residual carbonate content, e.g. by X-ray diffraction, or the slaking reactivity by common methods.

According to one embodiment of the present invention, the calcium oxide containing material of step i) is obtained by calcining a calcium carbonate containing material, preferably selected from the group consisting of precipitated calcium carbonate, natural calcium carbonate minerals such as marble, limestone and chalk, mixed alkaline earth carbonate minerals comprising calcium carbonate such as dolomite, and mixtures thereof.

For reasons of efficiency, it is preferred that the calcium oxide containing material has a minimum calcium oxide content of at least 75 wt.-%, preferably at least 90 wt.-%, and most preferably 95 wt.-%, based on the total weight of the calcium oxide containing material. According to one embodiment, the calcium oxide containing material consists of calcium oxide.

The calcium oxide containing material can consist of only one type of calcium oxide containing material. Alternatively, the calcium oxide containing material can consist of a mixture of two or more types of calcium oxide containing materials.

The calcium oxide containing material can be used in the inventive process in its original form, i.e. as a raw material, for example, in form of smaller and bigger chunks. Alternatively, the calcium oxide containing material can be ground before use. According to one embodiment of the present invention, the calcium carbonate containing material is in forms of particles having weight median particle size $d_{50}$ from 0.1 to 1 000 μm, and preferably from 1 to 500 μm.

Process step ii): provision of at least one depolymerized carboxylated cellulose

According to step ii) of the process of the present invention, at least one depolymerized carboxylated cellulose having a molecular weight $M_w$ in the range from 10 000 to 40 000 g/mol is provided.

A "carboxylated cellulose" in the meaning of the present invention is a cellulose that has been chemically modified and includes carboxylic units, i.e. at least a part of the hydroxyl groups of the original cellulose are carboxylated. Cellulose is a polysaccharide consisting of a linear chain of several hundred to many thousands of $\beta(1\rightarrow4)$ linked D-glucose units. The at least one carboxylated cellulose may have a degree of substitution of the hydroxyl groups in the range from 0.2 to 2, preferably from 0.4 to 1.8, more preferably from 0.5 to 1.6, and most preferably from 0.6 to 1.4.

The at least one carboxylated cellulose can be a carboxymethyl derivate and/or carboxymethyl hydroxypropyl derivate and/or carboxymethyl hydroxyethyl derivate of cellulose. According to a preferred embodiment of the present invention, the at least one modified polysaccharide is carboxymethylcellulose (CMC).

Carboxymethylcellulose (CMC) may be prepared from cellulose by reaction with monochloroacetic acid in the presence of caustic soda to form the sodium salt of carboxymethylcellulose. Each repeating D-glycose unit contains three hydroxyl groups theoretically capable of etherification, to give a theoretically maximum charge density of three carboxylic groups per monomer unit (i.e., a theoretically degree of substitution of three). The molecular weight of the carboxymethylcellulose can be adjusted by the treatment with hydrogen peroxide ($H_2O_2$). Reference is made to DE 1 543 116 A1 describing a method for the preparation of low viscous, water-soluble CMC by oxidative degradation with $H_2O_2$ (hydrogen peroxide) and to DE 44 11 681 A1 describing the dependency of the degradation of polysaccharide ether on the amount of oxidizing agent, temperature and duration of the treatment.

According to one embodiment of the present invention, the at least one depolymerized carboxylated cellulose has a molecular weight $M_w$ in the range from 13 000 to 35 000 g/mol, and most preferably in the range from 13 000 to 25 000 g/mol.

According to one embodiment of the present invention, the at least one depolymerized carboxylated cellulose has a polydispersity index from 2 to 10, preferably from 2 to 8, more preferably from 2.5 to 6, and most preferably from 3 to 5.

According to one embodiment of the present invention, the at least one depolymerized carboxylated cellulose may have a degree of substitution of the hydroxyl groups in the range from 0.2 to 2, preferably from 0.4 to 1.8, more preferably from 0.5 to 1.6, and most preferably from 0.6 to 1.4.

The at least one carboxylated cellulose may comprise one or more types of depolymerized carboxylated cellulose. According to one embodiment, only one type of depolymerized carboxylated cellulose is provided in process step ii). According to another embodiment, a mixture of at least two types depolymerized carboxylated cellulose is provided in process step ii).

The at least one depolymerized carboxylated cellulose can be provided as solution or dry material. For example, the at least one depolymerized carboxylated cellulose can be in form of an aqueous solution having a carboxylated cellulose concentration from 10 to 60 wt.-%, preferably from 25 to 45 wt.-%, more preferably from 30 to 40 wt.-%, and most preferably from 31 to 35 wt.-%, based on the total weight of the aqueous solution.

According to one embodiment of the present invention, the at least one depolymerized carboxylated cellulose has a Brookfield viscosity between 30 and 10 000 mPa·s at 25°C, preferably between 50 and 5000 mPa·s at 25°C, more preferably between 1000 and 3000 mPa·s at 25°C, and most preferably between 1500 and 2500 mPa·s at 25°C.

According to one embodiment of the present invention, the at least one depolymerized carboxylated cellulose is added in an amount from 0.001 to 5 wt.-%, preferably from 0.01 to 2 wt.-%, more preferably from 0.05 to 1 wt.-%, and most preferably from 0.1 to 0.5 wt.-%, based on the total weight of the calcium oxide containing material in the milk of lime. According to one embodiment of the present invention, the at least one depolymerized carboxylated cellulose is added in an amount such that the Brookfield viscosity of the aqueous suspension is between 60 and 2000 mPa·s at 25°C, and preferably between 80 and 700 mPa·s at 25°C.

According to one embodiment of the present invention, the at least one depolymerized carboxylated cellulose of the present invention has a pH from 4.5 to 12, preferably from 7 to 11, and more preferably from 8.0 to 10.5.

According to a preferred embodiment of the present invention, the at least one depolymerized carboxylated cellulose has a molecular weight $M_w$ in the range from 10 000 to 40 000 g/mol, preferably from 13 000 to 35 000 g/mol, and most preferably in the range from 13 000 to 25 000 g/mol, and is in form of an aqueous solution having a carboxylated cellulose concentration from 10 to 60 wt.-%, preferably from 25 to 45 wt.-%, more preferably from 30 to 40 wt.-%, and

most preferably from 31 to 35 wt.-%, based on the total amount of the aqueous solution. According to an exemplary embodiment, the at least one depolymerized carboxylated cellulose has a molecular weight $M_w$ in the range from 10 000 to 40 000 g/mol and is in form of an aqueous solution having a carboxylated cellulose concentration from 25 to 40 wt.-%, based on the total weight of the aqueous solution.

According to the present invention, the at least one depolymerized carboxylated cellulose is added during step iii) of the inventive process for producing PCC, i.e. the depolymerized carboxylated cellulose is added during the slaking step. As known to the skilled person, the milk of lime obtained by slaking a calcium oxide containing material with water has usually a pH value between 11 and 12.5 at a temperature of 25°C, depending on the concentration of the calcium oxide containing material in the milk of lime. Since the slaking reaction is exothermic, the temperature of the milk of lime typically raises to a temperature between 80 and 99°C. According to one embodiment of the present invention, the at least one depolymerized carboxylated cellulose of step ii) is selected such that it is stable in an aqueous suspension having a pH of 12 and a temperature of 95°C. In the meaning of the present invention, "stable in an aqueous suspension having a pH of 12 and a temperature of 95°C" means that the depolymerized carboxylated cellulose maintains its physical properties and chemical structure when added to an aqueous suspension having a pH of 12 and a temperature of 95°C. For example, the depolymerized carboxylated cellulose maintains its dispersing qualities and is not degraded under said conditions.

According to a preferred embodiment of the present invention, the at least one depolymerized carboxylated cellulose is a depolymerized carboxymethylcellulose (CMC) having has a molecular weight $M_w$ in the range from 10 000 to 40 000 g/mol. The depolymerized CMC may have a degree of carboxylation in the range from 0.2 to 2, preferably from 0.4 to 1.8, more preferably from 0.5 to 1.6, and most preferably from 0.6 to 1.4. According to another embodiment of the present invention, the depolymerized CMC comprises a blend of the two degrees of carboxylation, for example a blend of a carboxylation degree of about 0.8 and about 1.2.

According to another preferred embodiment of the present invention, the at least one depolymerized carboxylated cellulose is a depolymerized carboxymethylcellulose (CMC) having has a molecular weight $M_w$ in the range from 13 000 to 35 000 g/mol, preferably from 13 000 to 25 000 g/mol, a degree of carboxylation from 0.5 to 1.6, preferably from 0.6 to 1.4, and a polydispersity index from 2.5 to 6, preferably from 3 to 5. In addition, the least one depolymerized carboxymethylcellulose can be in form of an aqueous solution having a CMC concentration from 25 to 45 wt.-%, more preferably from 30 to 40 wt.-%, and most preferably from 31 to 35 wt.-%, based on the total amount of the aqueous solution.

The depolymerized carboxylated cellulose can be obtained by depolymerisation or degradation of a carboxylated cellulose having a molecular weight $M_w$ of more than 40 000 g/mol (measured by Gel Permeation, GPC), using any suitable method known to the skilled person.

According to one embodiment of the present invention, the depolymerized carboxylated cellulose is prepared by depolymerizing a high molecular weight carboxylated cellulose in a process comprising the following steps:

I) providing a high molecular weight carboxylated cellulose having a molecular weight $M_w$ of more than 40 000 g/mol and a degree of carboxylation in the range from 0.2 to 2,

II) providing a peroxide selected from hydrogen peroxide and/or an alkali metal salt thereof,

III) mixing the high molecular weight carboxylated cellulose of step I) and the peroxide of step II) and water incrementally and in any order at a reaction temperature from 50 to 85°C,

IV) maintaining the temperature of the mixture obtained from step III) until complete consumption of the peroxide,

V) cooling the mixture to a temperature of below 50°C, and

VI) optionally, neutralizing the obtained depolymerized carboxylated cellulose.

Preferably the carboxylated cellulose provided in step I) of the aforementioned process is carboxymethylcellulose, and thus, a depolymerized carboxymethylcellulose is prepared by process steps I) to VI).

The high molecular weight carboxylated cellulose can have a molecular weight $M_w$ from 50 000 to 700 000 g/mol, preferably from 100 000 to 500 000 g/mol, and more preferably from 200 000 to 400 000 g/mol. The high molecular weight carboxylated cellulose can have the same degree of carboxylation as the depolymerized carboxylated cellulose of process step ii) or can have a degree of carboxylation which is smaller than that of the depolymerized carboxylated cellulose. According to a preferred embodiment, the high molecular weight carboxylated cellulose has a molecular weight $M_w$ from 100 000 to 500 000 g/mol, preferably from 200 000 to 400 000 g/mol, and a degree of carboxylation from 0.5 to 1.6, preferably from 0.6 to 1.4.

According to one embodiment of the present invention, the peroxide is hydrogen peroxide. According to another embodiment the peroxide is an alkali metal peroxide, preferably sodium peroxide. According to another embodiment, the peroxide is a mixture of hydrogen peroxide and one or more alkali metal salts thereof. According to one embodiment of the present invention, the peroxide of step II) is provided in an amount from 0.1 to 50 wt.-%, preferably from 0.2 to 40 wt.-%, and more preferably from 1 to 30 wt.-%, based on the total amount of the high molecular weight carboxylated cellulose of step I). The peroxide can be provided in form of an aqueous solution having a concentration from 3 to 50

wt.-%, preferably from 25 to 40 wt.-%, based on the total amount of the aqueous solution.

The amount of the high molecular weight carboxylated cellulose of step I) and the peroxide of step II) is selected such that a depolymerized carboxylated cellulose is obtained having a molecular weight in the range of 10 000 to 40 000 g/mol and/or a polydispersity index in the range from 2 to 10.

In process step III), the high molecular weight carboxylated cellulose of step I), the peroxide of step II), and water may be mixed in any order. For example, at least one high molecular weight carboxylated cellulose can be mixed with water in a first step, and in a second step the peroxide can be added incrementally to the carboxylated cellulose/water mixture. Alternatively, the peroxide can be mixed with water in a first step, and in a second step the at least one high molecular weight carboxylated cellulose can added incrementally to the peroxide/water mixture. Alternatively, the least one high molecular weight carboxylated cellulose and the peroxide can be added incrementally and simultaneously to water.

According to one embodiment, a catalyst is added during step III). The catalyst may be selected from the group consisting of iron sulphate, sodium hypophosphate, iron phthalocyanine, sodium tungstate, and mixtures thereof. According to a further embodiment of the present invention, the catalyst is provided in an amount from 0.001 to 0.020 wt.-%, preferably from 0.002 to 0.015 wt.-%, and most preferably from 0.004 to 0.010 wt.-%, based on the total amount of the high molecular weight carboxylated cellulose of step I).

Process step III) can be carried out in a batch process or in a continuous process. A continuous process can be preferably carried out in a cascade mode of at least 2 vessels, preferably in a cascade mode of 2 to 10 vessels.

According to the present invention, during process step III) the Brookfield viscosity of the mixture is maintained between 200 and 1500 mPa·s, measured at the reaction temperature. The skilled person knows that the Brookfield viscosity of the mixture can be controlled by the amount of high molecular carboxylated cellulose that is added. For example, if a certain amount of high molecular weight carboxylated cellulose is mixed with the peroxide, the optional catalyst, and water, the Brookfield viscosity of the mixture will be reduced due to the depolymerisation reaction that will take place in presence of the peroxide and the optional catalyst. In order to maintain the Brookfield viscosity of the mixture in the desired range, further high molecular weight carboxylated cellulose may be added, which will increase the viscosity of the mixture. Furthermore, additional peroxide and, if necessary, catalyst can be added to the mixture to depolymerized the freshly added high molecular weight carboxylated cellulose. In case a mixture of peroxide, optional catalyst, and water is provided, high molecular weight carboxylated cellulose can be added to said mixture until the Brookfield viscosity of the mixture is in the desired range.

Furthermore, additional high molecular weight carboxylated cellulose, peroxide and, if necessary, catalyst can be added in a ratio to said mixture such that the Brookfield viscosity of the mixture is maintained in the desired range. According to one optional embodiment of the present invention, the viscosity of the mixture obtained in step III) is adjusted to a Brookfield viscosity between 200 and 1500 mPa·s, measured at the reaction temperature, preferably by adding a further high molecular weight carboxymethyl cellulose and/or a further peroxide and/or a further catalyst in one or more steps to the mixture obtained in step III).

According to one embodiment of the present invention, the final mixture obtained in step V) contains the depolymerized carboxylated cellulose of process step ii), i.e. a depolymerized carboxylated cellulose having a molecular weight in the range from 10 000 to 40 000 g/mol.

The depolymerized carboxylated cellulose mixture obtained by the aforementioned process steps I) to V) may be employed in the inventive process for producing an aqueous suspension of precipitated calcium carbonate without any further purification. According to an optional embodiment, the depolymerized carboxylated cellulose mixture obtained by the aforementioned process steps I) to V) is purified. Alternatively or additionally, the depolymerized carboxylated cellulose mixture obtained by the aforementioned process steps I) to V) can be diluted or concentrated.

The aforementioned depolymerization process of high molecular weight of carboxylated cellulose allows the direct preparation of depolymerized carboxylated cellulose solutions with a high concentration of carboxylated cellulose, and thus, energy consuming concentration steps such as thermal concentration or ultrafiltration can be avoided. Moreover, the obtained highly concentrated depolymerized carboxylated cellulose solution can be directly employed in the inventive process for producing an aqueous suspension of precipitated calcium carbonate.

Optionally, the process for preparing a depolymerized carboxylated cellulose comprises a step VI) of neutralizing the obtained depolymerized carboxylated cellulose.

According to one optional embodiment, the carboxylic groups of the depolymerized carboxylated cellulose are at least partly neutralized by one or more monovalent and/or polyvalent cations. According to a preferred embodiment, the monovalent cations are selected from $Li^+$, $Na^+$, $K^+$, or mixtures thereof. Preferably, the polyvalent cations are selected from $Sr^{2+}$, $Ca^{2+}$, $Mg^{2+}$, or mixtures thereof, and most preferably from $Ca^{2+}$ added in form of $Ca(OH)_2$ in suspension and/or solution. According to a preferred embodiment, the carboxylic groups of the depolymerized carboxylated cellulose are at least partly neutralized by $Ca^{2+}$ cations and the $Ca^{2+}$ is produced *in situ* by addition of a partially neutralized carboxylated cellulose and/or addition of an acid. Additionally or alternatively, the carboxylic groups of the depolymerized carboxylated cellulose are at least partly neutralized by one or more trivalent cations, preferably selected from $Al^{3+}$

and/or $Fe^{3+}$.

The monovalent cations and/or polyvalent cations can also be added during the preparation of the depolymerized carboxylated cellulose. For example, monovalent cations may be added during the optional neutralization of the depolymerized carboxylated cellulose in form of a base such as NaOH or KOH.

The monovalent cations may be added in the form of an aqueous salt solution, suspension or powder, and preferably in the form of a solution. The polyvalent cations may be added in the form of an aqueous salt solution, suspension or powder, and preferably in the form of a suspension.

The polyvalent cations may also be produced *in-situ,* e.g., by addition of an acid and/or acidic reacting salt and/or a partially neutralized carboxylated cellulose. The polyvalent cations may be added instead of monovalent cations or in combination with monovalent cations.

According to a preferred optional embodiment, the carboxylic groups of the depolymerized carboxylated cellulose are at least partly neutralized by adding prior and/or during and/or after process steps I) to V) one or more polyvalent cations, *in situ* formed, by adding an acid, preferably $H_3PO_4$, or acidic reacting salt, for example, $NaH_2PO_4$ preferably $CaHPO_4$.

The acid or acidic reacting salt may be added in an amount from 50 to 500 ppm, based on the total weight of the solids in the suspension, preferably in an amount from 200 to 400 ppm, preferably in the form of an aqueous solution or suspension.

It was found by the inventors that the addition of monovalent cations, and in particular the addition of polyvalent cations, to the suspension provides further advantages and especially provides improved adsorption properties of the depolymerized carboxylated cellulose to the surface of the forming precipitated calcium carbonate. This may enhance the effectiveness of the depolymerized carboxylated cellulose, and especially depolymerized carboxymethylcellulose, in the process of the present invention. The inventors of the present invention also found that the addition of a combination of monovalent cations and polyvalent cations may enhance the effectiveness of the depolymerized carboxylated cellulose in the inventive process particularly well.

According to one embodiment, one or more monovalent and/or one or more polyvalent cations are added in an amount from 0.1 to 5 wt.-%, preferably from 2 to 3 wt.-%, based on the total weight of the dry partially or fully neutralized salt of the depolymerized carboxylated cellulose. $Ca(OH)_2$ may be added in an amount from 50 to 500 ppm, based on the total weight of the mineral pigment material in the aqueous suspension, preferably from 200 to 300 ppm.

The depolymerized carboxylated cellulose can also be prepared by the methods described in EP 2 868 716 A1. Thus, the depolymerized carboxylated cellulose, preferably depolymerized carboxymethylcellulose (CMC), may be obtained by a process comprising the following steps:

A) providing a high molecular weight carboxylated cellulose, preferably a high molecular weight CMC, having a molecular weight of more than 40 000 g/Mol and a degree of carboxylation in the range from 0.2 to 2,
B) providing a peroxide selected from hydrogen peroxide and/or an alkali metal salt thereof,
C) providing a catalyst,
D) mixing at least one part of the high molecular weight carboxylated cellulose of step A) and at least one part of the peroxide of step B) and/or at least one part of the catalyst of step B) and water in any order at a reaction temperature from 50 to 85°C, and
E) adding the remaining part of the high molecular weight carboxylated cellulose and/or the remaining part of the peroxide and/or the remaining part of the catalyst in one or more steps to the mixture obtained in step D) until the mixture of step E) contains from 10 to 60 wt.-% depolymerized carboxylated cellulose, based on the total weight of the mixture of step E), and until the mixture of step E) at the same time has a Brookfield viscosity between 30 and 10 000 mPa·s at 20°C,

wherein during step E) the Brookfield viscosity of the mixture is maintained between 200 and 1500 mPa·s, measured at the reaction temperature.

The high molecular weight carboxylated cellulose, the peroxide, and the catalyst may be selected such as described above for the process comprising process steps I) to VI).

The amount of the high molecular weight carboxylated cellulose of step A), the peroxide of step B) and the catalyst of step B) may be selected such that a depolymerized carboxylated cellulose is obtained having a molecular weight in the range of 10 000 to 40 000 g/mol, and preferably a polydispersity index in the range from 2 to 10.

According to one embodiment, in process step D) at least one part of the high molecular weight carboxylated cellulose and at least one part of the peroxide and at least one part of the catalyst and water are mixed in any order. For example, the at least one part of the high molecular weight carboxylated cellulose can be mixed with water in a first step, and in a second step a mixture of the at least one part of the peroxide and the at least one part of the catalyst can be added to the carboxylated cellulose/water mixture. The at least one part of the peroxide and the at least one part of the catalyst can be added together to the carboxylated cellulose/water mixture or the at least one part of the catalyst

can be added in a first step to the carboxylated cellulose/water mixture, and the at least one part of the peroxide can be added in a second step to said mixture. Alternatively, the at least one part of the peroxide and the at least one part of the catalyst can be mixed with water in a first step, and in a second step the at least one part of the high molecular weight carboxylated cellulose can added to the peroxide/catalyst/water mixture. Alternatively, the at least one part of the high molecular weight carboxylated cellulose, the at least one part of the peroxide, and the at least one part of the catalyst can be mixed with water in one step.

According to another embodiment of the present invention, in process step D) the at least one part of the peroxide and the at least one part of the catalyst and water are mixed in any order. According to still another embodiment, in process step D) the at least one part of the high molecular weight carboxylated cellulose and the at least one part of the peroxide and water are mixed in any order. According to still another embodiment, in process step D) the at least one part of the high molecular weight carboxylated cellulose and the at least one part of the catalyst and water are mixed in any order. According to still another embodiment, in process step D) the at least one part of the catalyst and water are mixed.

According to the present invention, the expression "at least one part" means that a part of the provided compound or all of the provided compound is added. Accordingly, the expression "remaining part" refers to the part that is left over after the at least one part of the provided compound has been added. In the event that no part of the compound was added in process step D), the remaining part is all of the provided compound.

According to one embodiment, all of the provided peroxide is added in process step D). According to an alternative embodiment, 5%, 10%, 20%, 30%, 40% or 50% of the provided peroxide is added in process step D). According to still an alternative embodiment, all of the provided peroxide is added in process step E).

According to one embodiment, all of the provided catalyst is added in process step D). According to an alternative embodiment, 5%, 10%, 20%, 30%, 40% or 50% of the provided catalyst is added in process step D). According to still an alternative embodiment, all of the provided catalyst is added in process step E).

According to one embodiment, 5%, 10%, 20%, 30%, 40% or 50% of the provided high molecular weight carboxylated cellulose is added in process step D). According to an alternative embodiment, all of the provided high molecular weight carboxylated cellulose is added in process step E). In the event that at least one part of the high molecular weight carboxylated cellulose is added in process step D), the at least one part of the high molecular weight carboxylated cellulose may be selected such that the mixture obtained in step D) can be stirred. For example, the at least one part of the high molecular weight carboxylated cellulose may be selected such that the mixture obtained in step D) has a Brookfield viscosity between 200 and 1500 mPa·s measured at the reaction temperature.

According to one embodiment of the present invention, in step E) the remaining part of the high molecular weight carboxylated cellulose and/or the remaining part of the peroxide and/or the remaining part of the catalyst is/are added in one or more steps to the mixture obtained in step D) until the mixture of step E) contains from 25 to 45 wt.-% depolymerized carboxylated cellulose, preferably from 30 to 40 wt.-%, based on the total weight of the mixture of step E), and/or until the mixture of step E) has a Brookfield viscosity between 50 and 5000 mPa·s at 25°C, preferably between 1000 to 3000 mPa·s at 20°C, and most preferably between 1500 to 2500 mPa·s at 25°C.

According to one embodiment of the present invention, the remaining part of the high molecular weight carboxylated cellulose and/or the remaining part of the peroxide and/or the remaining part of the catalyst is/are added to the mixture obtained in step E) in 1 to 20 steps, preferably in 1 to 15 steps, more preferably in 2 to 12 steps, for example, in 3 to 5 or 10 to 12 steps.

According to one embodiment of the present invention, the remaining part of the high molecular weight carboxylated cellulose and/or the remaining part of the peroxide and/or the remaining part of the catalyst is/are added to the mixture obtained in step E) continuously. In other words, the high molecular weight carboxylated cellulose and/or the remaining part of the peroxide and/or the remaining part of the catalyst is/are added to the mixture obtained in step E) in small increments over a certain time period.

According to an exemplary embodiment of the present invention, the remaining part of the high molecular weight carboxylated cellulose is added in 2 to 12 steps and the remaining part of the peroxide is added continuously.

Process step E) can be carried out in a batch process or in a continuous process. A continuous process can be preferably carried out in a cascade mode of at least 2 vessels, preferably in a cascade mode of 2 to 10 vessels.

According to process step E) the Brookfield viscosity of the mixture is maintained between 200 and 1500 mPa·s, measured at the reaction temperature. The skilled person knows that the Brookfield viscosity of the mixture can be controlled by the amount of high molecular carboxylated cellulose that is added. For example, if a certain amount of high molecular weight carboxylated cellulose is mixed with the peroxide, the catalyst and water, the Brookfield viscosity of the mixture will be reduced due to the depolymerisation reaction that will take place in presence of the peroxide and the catalyst. In order to maintain the Brookfield viscosity of the mixture in the desired range, further high molecular weight carboxylated cellulose may be added, which will increase the viscosity of the mixture. Furthermore, additional peroxide and, if necessary, catalyst can be added to the mixture to depolymerized the freshly added high molecular weight carboxylated cellulose. In case a mixture of peroxide, catalyst and water is provided, high molecular weight carboxylated

cellulose can be added to said mixture until the Brookfield viscosity of the mixture is in the desired range. Furthermore, additional high molecular weight carboxylated cellulose, peroxide and, if necessary, catalyst can be added in a ratio to said mixture such that the Brookfield viscosity of the mixture is maintained in the desired range.

According to one optional embodiment, after step D) and before step E) the viscosity of the mixture obtained in step iv) is adjusted to a Brookfield viscosity between 200 and 1500 mPa·s, measured at the reaction temperature, preferably by adding a further part of the remaining part of the high molecular weight carboxylated cellulose and/or a further part of the remaining part of the peroxide and/or a further part of the remaining part of the catalyst in one or more steps to the mixture obtained in step D). According to one embodiment, 5%, 10%, 20%, 30%, 40% or 50% of the remaining part of the high molecular weight carboxylated cellulose and/or a the remaining part of the peroxide and/or a the remaining part of the catalyst is/are added in one or more steps to the mixture obtained in step D).

According to an optional embodiment, the mixture obtained in step E) is cooled to a temperature below 75°C. According to another optional embodiment, the mixture obtained in process step E) is neutralized. The neutralisation step may be carried out in the same way as described for process step VI) above.

According to one embodiment, the final mixture obtained in step E) contains a depolymerized carboxylated cellulose having a molecular weight in the range from 10 000 to 40 000 g/mol. The depolymerized carboxylated cellulose mixture obtained in process step E) may be employed in the aqueous suspension of the present invention without any further purification. According to an optional embodiment, the depolymerized carboxylated cellulose mixture obtained in process step E) is purified. Alternatively or additionally, the depolymerized carboxylated cellulose mixture obtained in process step v) can be diluted or concentrated.

Process step iii): preparation of the milk of lime

According to step iii) of the process of the present invention, a milk of lime is prepared by mixing water, the calcium oxide containing material of step i), and the at least one depolymerized carboxylated cellulose of step ii) to obtain a milk of lime, wherein the calcium oxide containing material and the water are mixed in a weight ratio from 1:1 to 1:12.

The reaction of the calcium oxide containing material with water results in the formation of a milky calcium hydroxide suspension, better known as milk of lime. Said reaction is highly exothermic and is also designated as "lime slaking" in the art.

According to one embodiment of the present invention, the temperature of the water, which is used in mixing step iii), i.e. the temperature of the water that is used to slake the calcium oxide containing material, is adjusted to be in the range from more than 0°C and less than 100°C. In other words, the water that is used to slake the calcium oxide containing material is adjusted to a temperature range, in which the water is in liquid form. Preferably, the temperature of the water, which is employed in mixing step iii) is adjusted to be from 1°C to 85°C, more preferably from 2°C to 70°C, even more preferably from 30°C to 65°C, and most preferably from 35 to 55°C. It will be apparent to the skilled person that the initial temperature of the water is not necessarily the same one as the temperature of the mixture prepared in step iii) due to the highly exothermic slaking reaction and/or due to the mixing of substances having different temperatures.

According to one embodiment of the present invention, process step iii) comprises the steps of:

a1) mixing the at least one depolymerized carboxylated cellulose of step ii) with water, and
a2) adding the calcium oxide containing material of step i) to the mixture of step a1).

According to one embodiment, step a1) is carried out at a temperature from more than 0°C to 99°C, preferably from 1°C to 75°C, more preferably from 2°C to 70°C, even more preferably from 30°C to 65°C, and most preferably from 35 to 55°C.

According to another embodiment of the present invention, process step iii) comprises the steps of:

b1) mixing the calcium oxide containing material of step i), and the at least one depolymerized carboxylated cellulose of step ii), and
b2) adding water to the mixture of step b1).

According to still another embodiment of the present invention, in process step iii) the calcium oxide containing material of step i), the at least one depolymerized carboxylated cellulose of step ii), and water are mixed simultaneously.

The at least one depolymerized carboxylated cellulose of step ii) may be added in step iii) in one portion or in several portions. According to one embodiment, in step iii) the at least one depolymerized carboxylated cellulose of step ii) is mixed with the water, and the calcium oxide containing material of step i), by adding the at least one depolymerized carboxylated cellulose in one portion or in two, three, four, five, or more portions.

Process step iii) may be performed at room temperature, i.e. at a temperature of 20°C ± 2°C, or at an initial temperature of 30 to 50°, preferably 35 to 45°C. Since the reaction is exothermic, the temperature typically raises to a

temperature between 85 and 99°C during step iii), preferably to a temperature between 90 and 95°C. According to a preferred embodiment, process step iii) is performed under mixing, agitation, or stirring, for example, mechanical stirring. Suitable process equipment for mixing, agitation or stirring is known to the skilled person.

The progress of the slaking reaction may be observed by measuring the temperature and/or conductivity of the reaction mixture. It can also be monitored by turbidity control. Alternatively or additionally, the progress of the slaking reaction can be inspected visually.

Conventional methods for preparing PCC suffer from the problem that the milk of lime can only be processed at low solids content since the milk of lime becomes very viscous at higher solids content during the slaking process. In a typical PCC production process of the prior art, the weight ratio of calcium oxide to water is less than 1:6, usually 1:9 or 1:10. The inventors surprisingly found that the addition of a depolymerized carboxylated cellulose as defined above, before or during the slaking step of a process for producing PCC can allow the preparation of a milk of lime with a high solids content. By carbonating said highly concentrated milk of lime, an aqueous suspension of PCC can be obtained which has also a high solids content. As a result, the process of the present invention does not require an additional up-concentration step in order to obtain a PCC suspension with a high solids content.

According to the present invention, the calcium oxide containing material and the water are mixed in a weight ratio from 1:1 to 1:12. According to one preferred embodiment, in step iii) the calcium oxide containing material and the water are mixed in a weight ratio from 1:1 to 1:9, more preferably from 1:2.5 to 1:5.

According to one embodiment of the present invention, the milk of lime of step iii) has a solids content of at least 8 wt.-%, preferably from 10 to 66 wt.-%, more preferably from 12 to 66 wt.-%, even more preferably from 15 to 55 wt.-%, and most preferably from 17 to 45 wt.-%, such as from 20 to 38 wt.-%, based on the total weight of the milk of lime.

According to one embodiment of the present invention, the milk of lime of step iii) has a Brookfield viscosity from 1 to 1 000 mPa·s at 25°C, more preferably from 5 and 800 mPa·s at 25°C, and most preferably from 10 and 500 mPa·s at 25°C. According to one embodiment, the Brookfield viscosity is measured at 100 rpm.

It is within the confines of the present invention that additional water may be introduced during the slaking reaction in order to control and/or maintain and/or achieve the desired solids content or Brookfield viscosity of the milk of lime.

Process step iii) can be carried out in form of a batch process, a semi-continuous or a continuous process. Fig. 1 shows an example of a continuous process step iii). The at least on carboxylated cellulose (2), the optional slaking additive (3), water (4), and a calcium oxide containing material (5) are fed into a slaker (1). The reaction heat (6) resulting from the exothermic slaking reaction is dissipated and the obtained milk of lime is discharged (7) to the next process stage, for example, the carbonation stage or a screening stage.

Process step iv): carbonation of the milk of lime

According to step iv) of the process of the present invention, the milk of lime obtained from step iii) is carbonated to form an aqueous suspension of precipitated calcium carbonate.

The carbonation is carried out by means and under conditions well-known by the person skilled in the art. The introduction of carbon dioxide into the milk of lime quickly increases the carbonate ion ($CO_3^{2-}$) concentration and calcium carbonate is formed. Particularly, the carbonation reaction can be readily controlled considering the reactions involved in the carbonation process. Carbon dioxide dissolves according to its partial pressure forming carbonate ions via the formation of carbonic acid ($H_2CO_3$), and hydrogen carbonate ions ($HCO_3^-$) being unstable in the alkaline solution. Upon continued dissolution of carbon dioxide, hydroxide ions are consumed and the concentration of carbonate ions increases until the concentration of dissolved calcium carbonate exceeds the solubility product and solid calcium carbonate precipitates.

According to one embodiment of the present invention, in step iv) the carbonation is carried out by feeding pure gaseous carbon dioxide or technical gases containing at least 10 vol.-% of carbon dioxide into the milk of lime.

The progress of the carbonation reaction can be readily observed by measuring the conductivity density, turbidity and/or pH. In this respect, the pH of the milk of lime before addition of carbon dioxide will be more than 10, usually between 11 and 12.5, and will constantly decrease until a pH of about 7 is reached. At this point the reaction can be stopped.

Conductivity slowly decreases during the carbonation reaction and rapidly decreases to low levels, when the precipitation is completed. The progress of the carbonation may be monitored by measuring the pH and/or the conductivity of the reaction mixture.

According to one embodiment of the present invention, the temperature of the milk of lime obtained from step iii), which is used in step iv) is adjusted to be in the range from 20°C to 60°C, and preferably from 30°C to 50°C. It will be apparent to the skilled person that the initial temperature of the milk of lime, is not necessarily the same one as the temperature of the mixture prepared in step iv) due to the exothermic carbonation reaction and/or due to the mixing of substances having different temperatures.

According to one embodiment of the present invention, step iv) is carried out at a temperature from 5 to 95°C,

preferably from 30 to 70°C, and more preferably from 40 to 60°C.

Process step iv) can be carried out in form of a batch process, a semi-continuous or a continuous process. According to one embodiment, the process of the present invention involving the process steps i) to iv) is carried out in form of a batch process, a semi-continuous or a continuous process.

According to one embodiment of the present invention, the process of the present invention does not comprise a step of up-concentrating the aqueous suspension of precipitated calcium carbonate obtained by steps i) to iv) of the inventive process. Additionally or alternatively, the process of the present invention does not comprise a step of separating the liquid phase from the solids content in the suspension obtained in step iii), i.e. there is no step of separating carried out between steps iii) and iv) of the inventive process.

As already mentioned above, the inventors surprisingly found that the addition of at least one depolymerized carboxylated cellulose as defined above before or during the slaking step of a process for producing PCC can allow the preparation of a PCC suspension with a high solids content. It is also believed that the omission of an up-concentration step improves the quality of the produced PCC particles, since surface damages of the particles, which can occur during the up-concentration step, are avoided. It was also found that said PCC suspension can be further up-concentrated to a solids contents of about 70 wt.-% at acceptable viscosities, for example, to Brookfield viscosities of less than or equal to 1 600 mPa·s at 25 °C and 100 rpm. Typically, this cannot be done with PCC suspensions that are obtained by conventional PCC production processes including an up-concentrating step because the viscosity of said suspension would raise to a non-pumpable range.

According to one embodiment of the present invention, the obtained precipitated calcium carbonate has a weight median particle size $d_{50}$ from 0.1 to 100 μm, preferably from 0.25 to 50 μm, more preferably from 0.3 to 5 μm, and most preferably from 0.4 to 3.0 μm.

The precipitated calcium carbonate may have aragonitic, calcitic, or vateritic crystal structure, or mixtures thereof. It is a further advantage of the present invention that the crystal structure and morphology of the precipitated calcium carbonate can be controlled, e.g. by addition of seed crystals or other structure modifying chemicals. According to a preferred embodiment, the precipitated calcium carbonate obtained by the inventive process has a clustered scalenohedral crystal structure.

The BET specific surface area of the precipitated calcium carbonate obtained by the process according to the present invention may be from 1 to 100 $m^2/g$, preferably from 2 to 70 $m^2/g$, more preferably from 3 to 50 $m^2/g$, especially from 4 to 30 $m^2/g$, measured using nitrogen and the BET method according to ISO 9277. The BET specific surface area of the precipitated calcium carbonate obtained by the process of the present invention may be controlled by the use of additives, e.g. surface active agents, shearing during the precipitation step or thereafter at high mechanical shearing rates not only leading to a low particle size, but also to a high BET specific surface area.

According to one embodiment of the present invention, the suspension of precipitated calcium carbonate obtained in step iv) has preferably a solids content of at least 10 wt.-%, preferably from 15 to 70 wt.-%, more preferably from 19 to 60 wt.-%, even more preferably from 21 to 50 wt.-% and most preferably from 24 to 42 wt.-%, based on the total weight of the suspension.

According to one embodiment of the present invention, the suspension of PCC of step iv) has a Brookfield viscosity of less than or equal to 1 600 mPa·s at 25°C, more preferably less than or equal to 1 500 mPa·s at 25°C, and most preferably less than or equal to 1 400 mPa·s at 25°C. The Brookfield viscosity is measured at 100 rpm.

Optional process steps

In one embodiment, the process further comprises step v) of adding at least one slaking additive to process step iii).

According to one embodiment of the present invention, the at least one slaking additive is selected from the group consisting of organic acids, organic acid salts, sugar alcohols, monosaccharides, disaccharides, polysaccharides, gluconates, phosphonates, lignosulfonates, and mixtures thereof.

For example, the at least one slaking additive is selected from the group consisting of sodium citrate, potassium citrate, calcium citrate, magnesium citrate, monosaccharides, disaccharides, polysaccharides, sucrose, sugar alcohols, meritol, citric acid, sorbitol, sodium salt of diethylene triamine pentaacetic acid, gluconates, phosphonates, sodium tartrate, sodium lignosulfonate, calcium lignosulfonate, and mixtures thereof. According to a preferred embodiment, the at least one slaking additive is sodium citrate and/or saccharose.

According to one embodiment of the present invention, the at least one slaking additive consists of one type of slaking additive only. Alternatively, the at least one slaking additive can consist of a mixture of two or more types of slaking additives.

The at least one slaking additive may be provided in an amount from 0.01 to 2.0 wt.-%, based on the total amount of calcium oxide containing material, preferably in an amount from 0.05 to 1.0 wt.-%, more preferably from 0.06 to 0.8 wt.-%, and most preferably from 0.07 to 0.5 wt.-%.

By adding a slaking additive, the size of the PCC particles and their crystal morphology can be controlled without affecting the viscosity of the aqueous suspension.

If the process of the present invention comprises a step of adding at least one slaking additive to process step iii), the process step iii) preferably comprises the steps of:

a1) mixing the at least one depolymerized carboxylated cellulose of step ii) and the at least one slaking additive with water, and
a2) adding the calcium oxide containing material of step i) to the mixture of step a1).

Alternatively, process step iii) comprises the steps of:

b1) mixing the calcium oxide containing material of step i), the at least one depolymerized carboxylated cellulose of step ii), and the at least one slaking additive, and
b2) adding water to the mixture of step b1).

Alternatively, in process step iii) the calcium oxide containing material of step i), the at least one depolymerized carboxylated cellulose of step ii), the at least one slaking additive, and water are mixed simultaneously.

According to still another embodiment of the present invention, the at least one slaking additive is added before or after step iii) of the inventive process.

In one embodiment of the present application, the milk of lime may be screened in order to remove oversize particles. A suitable screen can include, for example, a screen having a sieve size from 700 to 100 μm, for example, about 100 or about 300 μm. According to one embodiment of the present invention, the milk of lime is screened after step iii) and before step iv), preferably with a screen having a sieve size from 100 to 300 μm. It is to be noted that such a screening step is to be distinguished from a separating step as only particles of a specific size are removed. In contrast thereto, a separating step essentially completely removes the solids from an aqueous suspension.

It is possible to separate the precipitated calcium carbonate from the aqueous suspension obtained in step iv). In one embodiment, the process according to the present invention comprising the steps i) to iv), and optionally step v), thus further comprises step vi) of separating the precipitated calcium carbonate from the aqueous suspension obtained in step iv).

For the purpose of the present invention, the expression "separating" means that the PCC is removed or isolated from the aqueous suspension obtained from step iv) of the inventive process. The precipitated calcium carbonate obtained from step iv) may be separated from the mother liquor by any conventional means of separation known to the skilled person. According to one embodiment of the present invention, in process step vi) the PCC is separated mechanically and/or thermally. Examples for mechanical separation processes are filtration, e.g. by means of a drum filter or filter press, nanofiltration, or centrifugation. An example for a thermal separation process is an up-concentration process by the application of heat, for example, in an evaporator. According to a preferred embodiment, in process step vi) the PCC is separated mechanically, preferably by filtration and/or centrifugation.

It is also preferred that the mother liquor obtained after precipitation and/or any one of the reactants may be recycled into the process.

The obtained PCC may be further processed, e.g., may be deagglomerated or subjected to a dry grinding step. Otherwise, it may also be wet ground in form of a suspension. If the PCC is subjected to dewatering, dispersion and/or grinding steps, these steps may be accomplished by procedures known in the art. Wet grinding may be carried out in the absence of a grinding aid or in the presence of a grinding aid. One or more grinding agents can be included, such as, e.g., sodium polyacrylate, a salt of polyacrylate acid, and/or a salt of a copolymer of acrylic acid. Dispersants also can be included to prepare dispersions if desired.

In one embodiment, the separated precipitated calcium carbonate obtained from

step vi) is dried in drying step vii). Thus, a process for producing a precipitated calcium carbonate may comprise the steps of:

i) providing a calcium oxide containing material,
ii) providing at least one depolymerized carboxylated cellulose having a molecular weight $M_w$ in the range from 10 000 to 40 000 g/mol,
iii) preparing a milk of lime by mixing water, the calcium oxide containing material of step i), and the at least one depolymerized carboxylated cellulose of step ii) to obtain a milk of lime, wherein the calcium oxide containing material and the water are mixed in a weight ratio from 1:1 to 1:12,
iv) carbonating the milk of lime obtained in step iii) to form an aqueous suspension of precipitated calcium carbonate,
v) optionally adding at least one slaking additive to process step iii),
vi) separating the precipitated calcium carbonate form the aqueous suspension obtained in step iv), and

vii) drying the precipitated calcium carbonate obtained in step vi).

In general, the drying step vii) may take place using any suitable drying equipment and can, for example, include thermal drying and/or drying at reduced pressure using equipment such as an evaporator, a flash drier, an oven, a spray drier and/or drying in a vacuum chamber.

According to one embodiment, drying step vii) is a spray drying step, preferably said spray drying step is carried out at a lower temperature ranging from 90°C to 130°C and preferably from 100°C to 120°C. By means of drying step vii), a dried precipitated calcium carbonate is obtained having a low total moisture content which is less than or equal to 1.0 wt.-%, based on the total weight of the dried precipitated calcium carbonate.

According to another embodiment, the dried PCC of step vii) has a total moisture content of less than or equal to 0.5 wt.-% and preferably less than or equal to 0.2 wt.-%, based on the total weight of the dried precipitated calcium carbonate. According to still another embodiment, the dried PCC of step vii) has a total moisture content of between 0.01 and 0.15 wt.-%, preferably between 0.02 and 0.10 wt.-%, and more preferably between 0.03 and 0.07 wt.-%, based on the total weight of the dried precipitated calcium carbonate.

The precipitated calcium carbonate obtained by the inventive process can be post-treated, for example, during and/or after a drying step with an additional component.

According to one embodiment the process of the present invention further comprises a step viii) of contacting at least a part of the surface of the obtained precipitated calcium carbonate with at least one hydrophobising agent after step iv) and/or after step vi), if present, and/or during and/or after step vii), if present. Preferably, at least a part of the surface of the obtained precipitated calcium carbonate may be contacted with at least one hydrophobising agent during and/or after step vii).

Suitable hydrophobising agents are, for example, fatty acids, aliphatic carboxylic acids, aliphatic carboxylic esters, mono-substituted succinic anhydrides, mono-substituted succinic acids, or phosphoric acid esters. Suitable hydrophobising agents and methods for preparing surface-treated filler products thereof are, for example, described in EP 2 159 258 A1, EP 2 390 285 A1, EP 2 390 280 A1, WO 2014/060286 A1 and WO 2014/128087 A1.

In one embodiment, the hydrophobising agent is an aliphatic carboxylic acid having a total amount of carbon atoms from $C_4$ to $C_{24}$ and/or reaction products thereof. The term "reaction products" of the aliphatic carboxylic acid in the meaning of the present invention refers to products obtained by contacting the modified mineral-based filler with the at least one aliphatic carboxylic acid. Said reaction products are formed between at least a part of the at least one aliphatic carboxylic acid and reactive molecules located at the surface of the alkaline earth metal carbonate-comprising material particles.

The aliphatic carboxylic acid in the meaning of the present invention may be selected from one or more straight chain, branched chain, saturated, unsaturated and/or alicyclic carboxylic acids. Preferably, the aliphatic carboxylic acid is a monocarboxylic acid, i.e. the aliphatic carboxylic acid is characterized in that a single carboxyl group is present. Said carboxyl group is placed at the end of the carbon skeleton.

In one embodiment of the present invention, the aliphatic carboxylic acid is selected from saturated unbranched carboxylic acids, that is to say the aliphatic carboxylic acid is preferably selected from the group of carboxylic acids consisting of pentanoic acid, hexanoic acid, heptanoic acid, octanoic acid, nonanoic acid, decanoic acid, undecanoic acid, lauric acid, tridecanoic acid, myristic acid, pentadecanoic acid, palmitic acid, heptadecanoic acid, stearic acid, nonadecanoic acid, arachidic acid, heneicosylic acid, behenic acid, tricosylic acid, lignoceric acid and mixtures thereof.

In another embodiment of the present invention, the aliphatic carboxylic acid is selected from the group consisting of octanoic acid, decanoic acid, lauric acid, myristic acid, palmitic acid, stearic acid, arachidic acid and mixtures thereof. Preferably, the aliphatic carboxylic acid is selected from the group consisting of myristic acid, palmitic acid, stearic acid and mixtures thereof. For example, the aliphatic carboxylic acid is stearic acid.

Additionally or alternatively, the hydrophobising agent can be at least one mono-substituted succinic acid and/or salty reaction product(s) and/or at least one phosphoric acid ester blend of one or more phosphoric acid mono-ester and/or reaction products thereof and one or more phosphoric acid di-ester and/or reaction products thereof. Methods for treating a calcium carbonate-comprising material with these hydrophobising agents are described, for example, in EP 2 722 368 A1 and EP2770017A1.

The term "succinic anhydride", also called dihydro-2,5-furandione, succinic acid anhydride or succinyl oxide, has the molecular formula $C_4H_4O_3$ and is the acid anhydride of succinic acid. The term "mono-substituted succinic anhydride" in the meaning of the present invention refers to a succinic anhydride wherein a hydrogen atom is substituted by another substituent.

The term "reaction products of at least one mono-substituted succinic anhydride" in the meaning of the present invention refers to products obtained by contacting a alkaline earth metal carbonate-comprising material with one or more mono-substituted succinic anhydride(s). Said salty reaction products are formed between the mono-substituted succinic acid which is formed from the applied mono-substituted succinic anhydride and reactive molecules located at the surface of the alkaline earth metal carbonate-comprising material.

The term "phosphoric acid mono-ester" in the meaning of the present invention refers to an o-phosphoric acid molecule mono-esterified with one alcohol molecule selected from unsaturated or saturated, branched or linear, aliphatic or aromatic alcohols having a total amount of carbon atoms from $C_6$ to $C_{30}$, preferably from $C_8$ to $C_{22}$, more preferably from $C_8$ to $C_{20}$, and most preferably from $C_8$ to $C_{18}$ in the alcohol substituent. The term "phosphoric acid di-ester" in the meaning of the present invention refers to an o-phosphoric acid molecule di-esterified with two alcohol molecules selected from the same or different, unsaturated or saturated, branched or linear, aliphatic or aromatic alcohols having a total amount of carbon atoms from $C_6$ to $C_{30}$, preferably from $C_8$ to $C_{22}$, more preferably from $C_8$ to $C_{20}$, and most preferably from $C_8$ to $C_{18}$ in the alcohol substituent.

The term "salty reaction products of a phosphoric acid ester or blend of one or more phosphoric acid mono-esters and/or one or more phosphoric acid di-esters" in the meaning of the present invention refers to products obtained by contacting an alkaline earth metal carbonate-comprising material with one or more phosphoric acid mono-ester and one or more phosphoric acid di-ester and optionally phosphoric acid. Said salty reaction products are formed between the applied one or more phosphoric acid mono-ester and one or more phosphoric acid di-ester and optionally phosphoric acid and reactive molecules located at the surface of the alkaline earth metal carbonate-comprising material.

According to one embodiment, the at least one hydrophobising agent is selected from the group consisting of an aliphatic carboxylic acid having a total amount of carbon atoms from $C_4$ to $C_{24}$ and/or reaction products thereof, a mono-substituted succinic anhydride consisting of succinic anhydride mono-substituted with a group selected from a linear, branched, aliphatic and cyclic group having a total amount of carbon atoms from at least $C_2$ to $C_{30}$ in the substituent and/or reaction products thereof, a phosphoric acid ester blend of one or more phosphoric acid mono-ester and/or reaction products thereof and one or more phosphoric acid di-ester and/or reaction products thereof, polyhydrogensiloxane and reaction products thereof, an inert silicone oil, preferably polydimethylsiloxane, and mixtures thereof.

According to a preferred embodiment, the at least one hydrophobising agent is a mono-substituted succinic anhydride consisting of succinic anhydride mono-substituted with a group selected from a linear, branched, aliphatic and cyclic group having a total amount of carbon atoms from at least $C_2$ to $C_{30}$ in the substituent and/or a reaction product thereof

## Products and their use

According to one aspect not forming part of the invention, an aqueous suspension of precipitated calcium carbonate is provided, which is obtainable by a process comprising the steps of:

i) providing a calcium oxide containing material,
ii) providing at least one depolymerized carboxylated cellulose having a molecular weight $M_w$ in the range from 10 000 to 40 000 g/mol,
iii) preparing a milk of lime by mixing water, the calcium oxide containing material of step i), and the at least one depolymerized carboxylated cellulose of step ii) to obtain a milk of lime, wherein the calcium oxide containing material and the water are mixed in a weight ratio from 1:1 to 1:12, and
iv) carbonating the milk of lime obtained from step iii) to form an aqueous suspension of precipitated calcium carbonate, and
v) optionally, adding at least one slaking additive to process step iii).

According to a further aspect not forming part of the present invention, a precipitated calcium carbonate is provided, which is obtainable by a process comprising the steps of:

i) providing a calcium oxide containing material,
ii) providing at least one depolymerized carboxylated cellulose having a molecular weight $M_w$ in the range from 10 000 to 40 000 g/mol,
iii) preparing a milk of lime by mixing water, the calcium oxide containing material of step i), and the at least one depolymerized carboxylated cellulose of step ii) to obtain a milk of lime, wherein the calcium oxide containing material and the water are mixed in a weight ratio from 1:1 to 1:12,
iv) carbonating the milk of lime obtained from step iii) to form an aqueous suspension of precipitated calcium carbonate,
v) optionally, adding at least one slaking additive to process step iii), and
vi) separating the precipitated calcium carbonate from the aqueous suspension obtained from step iv).

Optionally, the obtained precipitated calcium carbonate can comprise a hydrophobising agent, which covers at least partially the surface of the precipitated calcium carbonate.

The PCC suspension and/or PCC obtained by the process of the present invention may be used in various

materials. For example, the precipitated calcium carbonate may be used in paper, plastics, polymer compositions, paint, coatings, concrete, cosmetics, pharmaceutics and/or agriculture applications. According to another example, the aqueous suspension of precipitated calcium carbonate may be used in paper, plastics, polymer compositions, paint, coatings, concrete, cosmetics, pharmaceutics and/or agriculture applications.

According to one aspect not forming part of the present invention, a product comprising the precipitated calcium carbonate according to the present invention is provided. For example, the product is a paper, a paper product, an ink, a paint, a coating, a plastic, a polymer composition, an adhesive, a building product, a foodstuff, an agricultural product, a cosmetic product or a pharmaceutical product.

According to still a further aspect not forming part of the present invention, a dried precipitated calcium carbonate is provided, which is obtainable by a process comprising the steps of:

i) providing a calcium oxide containing material,
ii) providing at least one depolymerized carboxylated cellulose having a molecular weight $M_w$ in the range from 10 000 to 40 000 g/mol,
iii) preparing a milk of lime by mixing water, the calcium oxide containing material of step i), and the at least one depolymerized carboxylated cellulose of step ii) to obtain a milk of lime, wherein the calcium oxide containing material and the water are mixed in a weight ratio from 1:1 to 1:12,
iv) carbonating the milk of lime obtained from step iii) to form an aqueous suspension of precipitated calcium carbonate,
v) optionally, adding at least one slaking additive to process step iii),
vi) separating the precipitated calcium carbonate from the aqueous suspension obtained from step iv), and
vii) drying the separated precipitated calcium carbonate obtained from step vi).

Optionally, the obtained dried precipitated calcium carbonate can comprise a hydrophobising agent, which covers at least partially the surface of the precipitated calcium carbonate.

According to one example, the dried precipitated calcium carbonate obtainable from process steps i) to vii) is a dried powder of precipitated calcium carbonate.

The dried PCC obtainable from process steps i) to vii) may be used in paper, plastics, polymer compositions, paint, coatings, concrete, cosmetics, pharmaceutics and/or agriculture applications. For example, the dried precipitated calcium carbonate is used in plastics and/or polymer compositions. For example, said PCC may be used in thermoplastic polymers, such as polyvinyl chloride, polyolefins, and polystyrene. Moreover, the dried PCC may also be used in polymer coatings which may be applied on the surface of polymer articles, such as foils, in order to increase the hydrophobicity (e.g., reflected by an increased contact angle measured against water) of said surface.

According to one aspect not forming part of the present invention, a product comprising dried precipitated calcium, preferably a dried powder of said precipitated calcium carbonate, is provided. For example, the product is a paper, a paper product, an ink, a paint, a coating, a plastic, a polymer composition, an adhesive, a building product, a foodstuff, an agricultural product, a cosmetic product or a pharmaceutical product. According to one example, a product comprising a dried precipitated calcium carbonate is provided, wherein the product is a plastic or a polymer composition.

The scope and interest of the present invention will be better understood based on the following figures and examples which are intended to illustrate certain embodiments of the present invention and are non-limitative.

Description of the figure:

Fig. 1 is a sketch of a continuous slaking process.

## Examples

1. Measurement methods

In the following, measurement methods implemented in the examples are described.

Brookfield viscosity

The Brookfield viscosity of the liquid coating compositions was measured after one hour of production and after one minute of stirring at 25°C ± 1°C at 100 rpm by the use of a Brookfield viscometer type RVT equipped with an appropriate disc spindle, for example spindle 2 to 5.

pH value

The pH of a suspension or solution was measured at 25°C using a Mettler Toledo Seven Easy pH meter and a Mettler Toledo InLab® Expert Pro pH electrode. A three point calibration (according to the segment method) of the instrument was first made using commercially available buffer solutions having pH values of 4, 7 and 10 at 20°C (from Sigma-Aldrich Corp., USA). The reported pH values are the endpoint values detected by the instrument (the endpoint was when the measured signal differed by less than 0.1 mV from the average over the last 6 seconds).

Particle size distribution

The particle size distribution of the prepared PCC particles was measured using a Sedigraph 5120 from the company Micromeritics, USA. The method and the instrument are known to the skilled person and are commonly used to determine grain size of fillers and pigments. The measurement was carried out in an aqueous solution comprising 0.1 wt.-% $Na_4P_2O_7$. The samples were dispersed using a high speed stirrer and supersonics. For the measurement of dispersed samples, no further dispersing agents were added.

Solids content of an aqueous suspension

The suspension solids content (also known as "dry weight") was determined using a Moisture Analyser MJ33 from the company Mettler-Toledo, Switzerland, with the following settings: drying temperature of 160°C, automatic switch off if the mass does not change more than 1 mg over a period of 30 sec, standard drying of 5 to 20 g of suspension.

Specific surface area (SSA)

The specific surface area was measured via the BET method according to ISO 9277 using nitrogen, following conditioning of the sample by heating at 250°C for a period of 30 minutes. Prior to such measurements, the sample was filtered within a Büchner funnel, rinsed with deionised water and dried overnight at 90 to 100°C in an oven. Subsequently the dry cake was ground thoroughly in a mortar and the resulting powder placed in a moisture balance at 130°C until a constant weight was reached.

Specific carbonation time

The monitoring of the conductivity, which slowly decreases during the carbonation reaction and rapidly decreases to a minimal level, thereby indicating the end of the reaction, was used to assess the time needed to perform the complete precipitation. The specific carbonation time (min/kg $Ca(OH)_2$) was determined by the following formula:

$$\text{Specific carbonation time} = \frac{10^5 \cdot Tf}{M \cdot SC_{MoL}}$$

wherein:

- *Tf* (min) is the time needed to complete the carbonation of the milk of lime, as determined by monitoring the conductivity,
- *M* (g) is the weight of the milk of lime introduced into the carbonation reactor, and
- $SC_{MoL}$ (%) is the weight solids content of the milk of lime.

Charge measurement - Mütek

The charge measurement was carried out using a Mütek PCD 03 device equipped with a Mütek PCD titrator.

About 1 g of the PCC suspension is weighed in the plastic measuring cell and is diluted with 20 mL of deionised water. Put the displacement piston on. While the piston oscillates in the cell, wait until the streaming current between the two electrodes stabilize.

The sign of the measured value shown on the display indicates whether the charge of the sample is positive (cationic) or negative (anionic). An oppositely charged polyelectrolyte of known charge density is added to the sample as a titrant (either sodium polyoxyethylene sulfate 0.001 N or pDADMAC 0.001 N). The titrant charges neutralize existing charges of the sample. Titration is discontinued as soon as the point of zero charge (0 mV) is reached.

Titrant consumption in mL forms the basis for further calculations. The specific charge quantity q [μVal/g of sly] is calculated according to the following formula:

$$q = (V \text{ x } c) / m$$

V: consumed titrant volume [l]
c: titrant concentration [μVal/l]
m: mass of the weighed slurry [g]
q: specific charge quantity [μVal/g of slurry]

Degree of carboxylation

The degree of carboxylation was determined by conductometric titration according to Katz et al. "The determination of strong and weak acidic groups in sulfite pulps" (Svensk Paperstidn., 1984, 6, pp. 48-53).

Molecular weight $M_w$ and $M_n$

The molecular weight $M_w$ of the carboxylated cellulose was determined by gel permeation chromatography (GPC) using a Waters™ liquid chromatograph with a Waters™ refractometric detector.

The mobile phase was a 1 N sodium hydroxide solution adjusted to pH 9 and containing 0.05 mol/l $NaHCO_3$, 0.1 mol/l $NaNO_3$, 0.02 mol/l triethanolamine, and 0.03 wt.-% of $NaN_3$.

In a first step, the solution of carboxylated cellulose was diluted to a concentration of 0.9 wt.-%, based on the total weight of the solution, with a solvent which corresponded to the mobile phase, but additionally contained 0.04% dimethylformamide as flow rate marker or internal standard. Subsequently, the solution was filtered with a 0.2 μm filter and 100 μl of the filtered solution were injected into the GPC (mobile phase: 1 N sodium hydroxide solution adjusted to pH 9 and containing 0.05 mol/l $NaHCO_3$, 0.1 mol/l $NaNO_3$, 0.02 mol/l triethanolamine, and 0.03 wt.-% of $NaN_3$).

The liquid chromatography apparatus contained an isocratic pump of the Waters™ 515 type, the flow rate of which was set at 0.8 ml/min, a Waters™ 717+ sample changer, a kiln containing a precolumn of the "Guard Column Ultrahydrogel Waters™" type which was 6 cm in length and had an internal diameter of 40 mm, followed by a linear column of the "Ultrahydrogel Waters™" type which was 30 cm in length and had an internal diameter of 7.8 mm. Detection was accomplished by means of a Waters™ 410 type differential refractometer. The kiln was heated to a temperature of 60°C and the refractometer was heated to a temperature of 45°C.

The liquid chromatography apparatus was calibrated with a series of certified sodium polyacrylate standards of differend molecular weights, supplied by Polymer Standard Service or American Standard Polymer Corporation.

The calibration graph is of the linear type and takes into account the correction obtained using the flow rate marker dimethylformamide. Acquisition and processing of the chromatogram were accomplished through the use of the PSS WinGPC Scientific v. 4.02 application. The chromatogram obtained was integrated in the area corresponding to molecular weights higher than 65 g/mol.

Polydispersity index (DPI)

The polydispersity index of a polymer is the ratio of the mass-average molecular weight in weight $M_w$ to the number-average molecular weight $M_n$. Both $M_w$ and $M_n$ were determined by gel permeation chromatography.

X-ray diffraction

The purity and morphology of the PCC samples was analysed with a D8 Advance powder diffractometer (Bruker Corporation, USA) obeying Bragg's law. This diffractometer consisted of a 2.2 kW X-ray tube (Cu), a sample holder, a $\vartheta$-$\vartheta$ goniometer, and a VANTEC-1 detector. Nickel-filtered Cu $K_\alpha$ radiation was employed in all experiments ($\lambda K_{\alpha\text{-Cu}}$ = 1.5406 Å). The profiles were chart recorded automatically using a scan speed of 0.7° per minute in 2$\vartheta$ (XRD GV_7600). The measurement was carried out at angles from 5 to 70°.

The resulting powder diffraction pattern was classified by mineral content using the DIFFRAC$^{suite}$ software packages EVA and SEARCH, based on reference patterns of the ICDD PDF 2 database (XRD LTM_7603). Quantitative analysis of the diffraction data, i.e. the determination of amounts of different phases in a multi-phase sample, has been performed using the DIFFRAC$^{suite}$ software package TOPAS (XRD LTM_7604). This involved modelling the full diffraction pattern (Rietveld approach) such that the calculated pattern(s) duplicated the experimental one.

## 2. Polymer additives and slaking additives

CMC: High molecular weight carboxymethylcellulose ($M_w$= 250 000 g/mol, carboxylation degree = 1.2), commercially available from Sigma-Aldrich, Switzerland, reference 419281.

SA1: Sodium citrate, commercially available from Sigma-Aldrich, Switzerland.

PA1: Depolymerized carboxymethylcellulose, produced according to Example 1.

PA2: High molecular weight carboxymethylcellulose Blanose® ($M_w$= 395 000 g/mol, carboxylation degree = 1.2), commercially available from Ashland Inc., USA.

PA3: polyacrylic acid with the following formula,

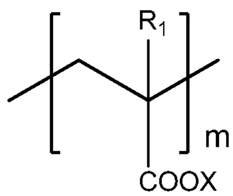

wherein $R_1$ is H, X is Na, and m = 45; the $M_w$ being 4270 g/mol, and the polydispersity index being 2.3. The molecular weight $M_w$ and the polydispersity index were determined according to the corresponding method described in EP 14 166 751.9.

## 3. Examples

Example 1 - Preparation of depolymerized carboxymethylcellulose

A one litre reactor was charged with 800 g distilled water and 0.017 g of catalyst $FeSO_4 \cdot 7\ H_2O$. The reactor was heated to 80°C±2°C. Over a time period of 2 hours and 45 minutes, an aqueous hydrogen peroxide solution having a concentration of 35 wt.-% was continuously added at a rate of 189 mg/min, while CMC was added in portions of 25 g at a time every 15 minutes. After completed addition, the reaction mixture was maintained at 80°C for 2 hours and 30 minutes until all hydrogen peroxide was consumed. Subsequently, the reaction mixture was cooled down to 70°C.

The pH of the obtained reaction mixture was 4.4. The reaction mixture was neutralized to pH 7.4 with an aqueous solution containing 10 wt.-% sodium hydroxide, based on the total amount of the solution.

The obtained depolymerized CMC had a $M_w$ of 13 310 g/mol, a polydispersity index of 4, and was in form of a solution having a concentration of 33.0 wt.-%, based on the total amount of the solution, and a Brookfield viscosity of 725 mPa·s at 25°C and 100 rpm.

Example 2 - Preparation of PCC (Samples 1 to 5)

A milk of lime was prepared by mixing under mechanical stirring water with 0.1 wt.-%, based on the total amount of calcium oxide, dry sodium citrate (SA1) as slaking additive and 0.15 wt.-%, based on the total amount of calcium oxide, of the depolymerized CMC produced according to Example 1 (PA21) or one of the other polymer additives indicated in Table 1 below (PA2 or PA3), at an initial temperature between 40 and 41°C. Subsequently, calcium oxide (quicklime raw material) was added. The obtained mixture was stirred for 25 min and then sieved through a 200 μm screen.

The obtained milk of lime was transferred into a stainless steel reactor, wherein the milk of lime was cooled down to 50°C. Then the milk of lime was carbonated by introducing an air/$CO_2$ mixture (26 vol-% $CO_2$). During the carbonation step, the reaction mixture was stirred with a speed of 1400 rpm. The kinetic of the reaction was monitored by online pH and conductivity measurements.

In addition two comparative example were produced without depolymerized carboxymethyl cellulose or other polymer additives.

The characteristics of the prepared milks of lime and aqueous PCC suspensions are compiled in Table 1 below.

Table 1: Characteristics of the produced milks of lime and the obtained aqueous PCC suspensions of Example 2 (comp.: comparative, nm: not measured).

| | Sample 1 (comp.) | Sample 2 (comp.) | Sample 3 (inventive) | Sample 4 (comp.) | Sample 5 (comp.) |
|---|---|---|---|---|---|
| Polymer additive | -- | -- | PA1 | PA2 | PA3 |
| Solids content of milk of lime [wt.-%] | 25.1 | 16.2 | 28.1 | 28.1 | 29.5 |
| Brookfield viscosity of milk of lime [mPa·s] | too high | 23 | 410 | too high | 329 |
| Carbonation time [min/kg $Ca(OH)_2$] | nm | 44.2 | 47 | nm | 46.5 |
| Solids content of PCC suspension [wt.-%] | nm | 20.5 | 36.6 | nm | 37.6 |
| $d_{50}$ [μm] | nm | 1.6 | 1.5 | nm | 1.3 |
| SSA [$m^2/g$] | nm | 4.7 | 4.7 | nm | 5 |
| pH | nm | nm | 7.9 | nm | nm |
| Brookfield viscosity of PCC suspension [mPa·s] | nm | 20 | 597 | nm | 940 |
| Mütek [μVal/g) | nm | 0.1 | -0.5 | nm | -0.9 |

The results presented in Table 1 above confirm that a PCC suspension with a high solids content can be produced by using the process of the present invention (sample 3). In contrast, it was not possible to produce a PCC suspension with a high solids content by carrying out the aforementioned process in the absence of a depolymerized cellulose (see comparative sample 1). The use of carboxymethylcellulose (PA2) instead of depolymerized carboxymethylcellulose (PA21) also resulted in a milk of lime having such a high Brookfield viscosity (above 1000 mPa·s at 25°C ± 1°C at 100 rpm) that a further processing of the sample was impossible (see comparative sample 4).

Example 3 - Preparation of PCC (Samples 6 and 7)

*Inventive sample 6*

A milk of lime was prepared by mixing under mechanical stirring 9 l water with 0.1 wt.-%, based on the total amount of calcium oxide, dry sodium citrate (SA1) as slaking additive and 0.15 wt.-%, based on the total amount of calcium oxide, of the depolymerized CMC produced according to Example 1 (PA21) at an initial temperature between 40 and 41°C. Subsequently, calcium oxide (quicklime raw material) was added, wherein the calcium oxide/water ratio was adjusted to 1: 4.4-3.9 in order to obtain a milk of lime with a high solids content and an acceptable Brookfield viscosity of up to 350-400 mPa·s. The obtained mixture was stirred for 25 min and then sieved through a 200 μm screen.

8 1 of the obtained milk of lime were transferred into a stainless steel reactor, wherein the milk of lime was cooled down to 50°C. Then the milk of lime was carbonated by introducing an air/$CO_2$ mixture (20 vol-% $CO_2$) with a rate of 15 l/min. During the carbonation step, the reaction mixture was stirred with a speed of 750 rpm. The kinetic of the reaction was monitored by online pH and conductivity measurements.

*Comparative sample 7*

A milk of lime was prepared by mixing under mechanical stirring 9 l water with 0.1 wt.-%, based on the total amount of calcium oxide, dry sodium citrate (SA1) as slaking additive. Subsequently, calcium oxide (quicklime raw material) was added in an amount such that a solids content of 13.5 wt.-%, based the total amount of the milk of lime, was obtained. The obtained mixture was stirred for 25 min and then sieved through a 200 μm screen.

8 1 of the obtained milk of lime were transferred into a stainless steel reactor, wherein the milk of lime was cooled down to 50°C. Then the milk of lime was carbonated by introducing an air/$CO_2$ mixture (20 vol-% $CO_2$) with a rate of 15 l/min. During the carbonation step, the reaction mixture was stirred with a speed of 750 rpm. The kinetic of

the reaction was monitored by online pH and conductivity measurements.

The obtained PCC suspension had a solids content of 17.5 wt.-%, based on the total amount of the suspension, and was mechanically up-concentrated to a solids content of 35.8 wt.-%.

The characteristics of the prepared milks of lime and aqueous PCC suspensions are compiled in Table 2 below.

Table 2: Characteristics of the produced milk of lime and the obtained aqueous PCC suspension of Example 3 (*after up-concentration).

| | Sample 6 (inventive) | Sample 7 (comparative) |
|---|---|---|
| Solids content of milk of lime [wt.-%] | 28.5 | 13.5 |
| Brookfield viscosity of milk of lime [mPa·s] | 200 | 30 |
| Solids content of PCC suspension [wt.-%] | 33.6 | 17.5 (35.8*) |
| $d_{50}$ [μm] | 1.95 | 2.37 |
| SSA [$m^2/g$] | 5.7 | 6.1 |
| pH | 8.5 | 10 |
| Brookfield viscosity of PCC suspension [mPa·s] | 285 | 270* |
| morphology | S-PCC | S-PCC |

As can be seen from the results shown in Table 2 above, a PCC suspension with a high solids content has been produced by using the process of the present invention (sample 6). Furthermore, the precipitated calcium carbonate (PCC) produced by the process of the present invention (sample 6) features a substantially lower average particle size, and thus, a higher fineness, compared to the PCC obtained by the prior art process (sample 7).

## Claims

**1.** A process for producing an aqueous suspension of precipitated calcium carbonate comprising the steps of:

i) providing a calcium oxide containing material,
ii) providing at least one depolymerized carboxylated cellulose having a molecular weight $M_w$ in the range from 10 000 to 40 000 g/mol,
iii) preparing a milk of lime by mixing water, the calcium oxide containing material of step i), and the at least one depolymerized carboxylated cellulose of step ii) to obtain a milk of lime, wherein the calcium oxide containing material and the water are mixed in a weight ratio from 1:1 to 1:12, and
iv) carbonating the milk of lime obtained in step iii) to form an aqueous suspension of precipitated calcium carbonate.

**2.** The process of claim 1, wherein step iii) comprises the steps of:

a1) mixing the at least one depolymerized carboxylated cellulose of step ii) with water, and a2) adding the calcium oxide containing material of step i) to the mixture of step a1), or
b1) mixing the calcium oxide containing material of step i), and the at least one depolymerized carboxylated cellulose of step ii), and b2) adding water to the mixture of step b1), or
c) mixing the calcium oxide containing material of step i), the at least one depolymerized carboxylated cellulose of step ii) and water simultaneously.

**3.** The process of any one of the previous claims, wherein the process further comprises step v) of adding at least one slaking additive to process step iii), preferably the at least one slaking additive is selected from the group consisting of organic acids, organic acid salts, sugar alcohols, monosaccharides, disaccharides, polysaccharides, gluconates, phosphonates, lignosulfonates, and mixtures thereof.

4. The process of any one of the previous claims, wherein
the milk of lime obtained in step iii) has a Brookfield viscosity from 1 to 1000 mPa·s at 25°C, more preferably from 5 and 800 mPa·s at 25°C, and most preferably from 10 and 500 mPa·s at 25°C, and/or
the suspension of PCC obtained in step iv) has a Brookfield viscosity of less than or equal to 1600 mPa·s at 25°C, more preferably less than or equal to 1500 mPa·s at 25°C, and most preferably less than or equal to 1400 mPa·s at 25°C.

5. The process of any one of the previous claims, wherein the suspension of PCC obtained in step iv) has a solids content of at least 10 wt.-%, preferably from 15 to 70 wt.-%, more preferably from 19 to 60 wt.-%, even more preferably from 21 to 50 wt.-%, and most preferably from 24 to 42 wt.-%, based on the total amount of the suspension.

6. The process of any one of the previous claims, wherein the depolymerized carboxylated cellulose has a polydispersity index from 2 to 10, preferably from 2 to 8, more preferably from 2.5 to 6, and most preferably from 3 to 5.

7. The process of any one of the previous claims, wherein the depolymerized carboxylated cellulose has degree of carboxylation from 0.2 to 2, preferably from 0.4 to 1.8, more preferably from 0.5 to 1.6, and most preferably from 0.6 to 1.4.

8. The process of any one of the previous claims, wherein the depolymerized carboxylated cellulose has molecular weight $M_w$ in the in the range from 13 000 to 35 000 g/mol, and preferably in the range from 13 000 to 25 000 g/mol.

9. The process of any one of the previous claims, wherein the depolymerized carboxylated cellulose
is provided in form of a solution having a solids content from 10 to 60 wt.-%, based on the total weight of the solution, preferably from 25 to 45 wt.-%, more preferably from 30 to 40 wt.-%, and most preferably from 31 to 35 wt.-%, and/or
is added in an amount from 0.001 to 5 wt.-%, based on the total weight of the calcium oxide containing material in the milk of lime, preferably from 0.01 to 2 wt.-%, more preferably from 0.05 to 1 wt.-%, and most preferably from 0.1 to 0.5 wt.-%.

10. The process of any one the previous claims, wherein the depolymerized carboxylated cellulose is prepared by depolymerizing a high molecular weight carboxylated cellulose in a process comprising the following steps:

I) providing a high molecular weight carboxylated cellulose having a molecular weight of more than 40 000 g/mol and a degree of carboxylation in the range from 0.2 to 2,
II) providing a peroxide selected from hydrogen peroxide and/or an alkali metal salt thereof,
III) mixing the high molecular weight carboxylated cellulose of step I) and the peroxide of step II) and water incrementally and in any order at a reaction temperature from 50 to 85°C,
IV) maintaining the temperature of the mixture obtained from step III) until complete consumption of the peroxide,
V) cooling the mixture to a temperature of below 50°C, and
VI) optionally, neutralizing the obtained depolymerized carboxylated cellulose.

11. The process of any one of the previous claims, wherein the depolymerized carboxylated cellulose is a carboxymethyl derivate and/or carboxymethyl hydroxypropyl derivate and/or carboxymethyl hydroxyethyl derivate of cellulose, preferably the depolymerized carboxylated cellulose is depolymerized carboxymethylcellulose.

12. The process of any one of the previous claims, wherein the process further comprises step vi) of separating the precipitated calcium carbonate from the aqueous suspension obtained in step iv), and optionally step vii) of drying the separated precipitated calcium carbonate obtained in step vi).

13. The process of any one of the previous claims, wherein the process further comprises a step viii) of contacting at least a part of the surface of the obtained precipitated calcium carbonate with at least one hydrophobising agent after step iv) and/or after step vi), if present, and/or during and/or after step vii), if present, preferably the at least one hydrophobising agent is selected from the group consisting of an aliphatic carboxylic acid having a total amount of carbon atoms from $C_4$ to $C_{24}$ and/or reaction products thereof, a mono-substituted succinic anhydride consisting of succinic anhydride mono-substituted with a group selected from a linear, branched, aliphatic and cyclic group having a total amount of carbon atoms from at least $C_2$ to $C_{30}$ in the substituent and/or reaction products thereof, a phosphoric acid ester blend of one or more phosphoric acid mono-ester and/or reaction products thereof and one or more phosphoric acid di-ester and/or reaction products thereof, polyhydrogensiloxane and reaction products thereof, an inert silicone oil, preferably polydimethylsiloxane, and mixtures thereof.

## Patentansprüche

1. Verfahren zur Herstellung einer wässrigen Suspension von gefälltem Calciumcarbonat, umfassend die Schritte:

   i) Bereitstellen von einem Calciumoxid enthaltenden Material,
   ii) Bereitstellen von wenigstens einer depolymerisierten carboxylierten Cellulose mit einem Molekulargewicht $M_w$ im Bereich von 10 000 bis 40 000 g/mol,
   iii) Zubereiten von Kalkmilch durch Vermischen von Wasser, dem Calciumoxid enthaltenden Material aus Schritt i) und der wenigstens einen depolymerisierten carboxylierten Cellulose aus Schritt ii) unter Erhalt von Kalkmilch, wobei das Calciumoxid enthaltende Material und das Wasser in einem Gewichtsverhältnis von 1:1 bis 1:12 vermischt werden, und
   iv) Carbonisieren der in Schritt iii) erhaltenen Kalkmilch unter Bildung einer wässrigen Suspension von gefälltem Calciumcarbonat.

2. Verfahren nach Anspruch 1, bei dem Schritt iii) die Schritte umfasst:

   a1) Vermischen der wenigstens einen depolymerisierten carboxylierten Cellulose aus Schritt ii) mit Wasser, und a2) Zugeben des Calciumoxid enthaltenden Materials aus Schritt i) zu dem Gemisch aus Schritt a1), oder
   b1) Vermischen des Calciumoxid enthaltenden Materials aus Schritt i) und der wenigstens einen depolymerisierten carboxylierten Cellulose aus Schritt ii), und b2) Zugeben von Wasser zu dem Gemisch aus Schritt b1), oder
   c) gleichzeitiges Vermischen des Calciumoxid enthaltenden Materials aus Schritt i), der wenigstens einen depolymerisierten carboxylierten Cellulose aus Schritt ii) und des Wassers.

3. Verfahren nach einem der vorhergehenden Ansprüche, bei dem das Verfahren ferner Schritt v) umfasst, bei dem wenigstens ein Löschadditiv zu Verfahrensschritt iii) zugegeben wird, bevorzugt ist das wenigstens eine Löschadditiv ausgewählt aus der Gruppe bestehend aus organischen Säuren, organischen Säuresalzen, Zuckeralkoholen, Monosacchariden, Disacchariden, Polysacchariden, Gluconaten, Phosphonaten, Lignosulfonaten, sowie Gemischen davon.

4. Verfahren nach einem der vorhergehenden Ansprüche, bei dem die in Schritt iii) erhaltene Kalkmilch eine Brookfield-Viskosität von 1 bis 1 000 mPa·s bei 25°C aufweist, bevorzugt von 5 bis 800 mPa·s bei 25°C, und am meisten bevorzugt von 10 bis 500 mPa·s bei 25°C, und/oder
   die in Schritt iv) erhaltene Suspension von PCC weist eine Brookfield-Viskosität von kleiner oder gleich 1 600 mPa·s bei 25°C auf, bevorzugter kleiner oder gleich 1 500 mPa·s bei 25°C, und am meisten bevorzugt kleiner oder gleich 1 400 mPa·s bei 25°C.

5. Verfahren nach einem der vorhergehenden Ansprüche, bei dem die in Schritt iv) erhaltene Suspension von PCC einen Feststoffgehalt von wenigstens 10 Gew.-% aufweist, bevorzugt von 15 bis 70 Gew.-%, bevorzugter von 19 bis 60 Gew.-%, noch bevorzugter von 21 bis 50 Gew.-%, und am meisten bevorzugt von 24 bis 42 Gew.-%, bezogen auf das Gesamtgewicht der Suspension.

6. Verfahren nach einem der vorhergehenden Ansprüche, bei dem die depolymerisierte carboxylierte Cellulose einen Polydispersitätsindex von 2 bis 10 aufweist, bevorzugt von 2 bis 8, bevorzugter von 2,5 bis 6, und am meisten bevorzugt von 3 bis 5.

7. Verfahren nach einem der vorhergehenden Ansprüche, bei dem die depolymerisierte carboxylierte Cellulose einen Carboxylierungsgrad von 0,2 bis 2 aufweist, bevorzugt von 0,4 bis 1,8, bevorzugter von 0,5 bis 1,6, und am meisten bevorzugt von 0,6 bis 1,4.

8. Verfahren nach einem der vorhergehenden Ansprüche, bei dem die depolymerisierte carboxylierte Cellulose ein Molekulargewicht $M_w$ im Bereich von 13 000 bis 35 000 g/mol aufweist, und bevorzugt im Bereich von 13 000 bis 25 000 g/mol.

9. Verfahren nach einem der vorhergehenden Ansprüche, bei dem die depolymerisierte carboxylierte Cellulose in der Form einer Lösung mit einem Feststoffgehalt von 10 bis 60 Gew.-%, bezogen auf das Gesamtgewicht der Lösung, bereitgestellt wird, bevorzugt von 25 bis 45 Gew.-%, bevorzugter von 30 bis 40 Gew.-%, und am meisten bevorzugt von 31 bis 35 Gew.-%, und/oder

in einer Menge von 0,001 bis 5 Gew.-%, bezogen auf das Gesamtgewicht des Calciumoxid enthaltenden Materials in der Kalkmilch, zugegeben wird, bevorzugt von 0,01 bis 2 Gew.-%, bevorzugter von 0,05 bis 1 Gew.-%, und am meisten bevorzugt von 0,1 bis 0,5 Gew.-%.

**10.** Verfahren nach einem der vorhergehenden Ansprüche, bei dem die depolymerisierte carboxylierte Cellulose hergestellt wird durch Depolymerisieren einer carboxylierten Cellulose mit hohem Molekulargewicht in einem Verfahren umfassend die folgenden Schritte:

I) Bereitstellen von einer carboxylierten Cellulose mit hohem Molekulargewicht mit einem Molekulargewicht von mehr als 40 000 g/mol und einem Carboxylierungsgrad im Bereich von 0,2 bis 2,
II) Bereitstellen von einem Peroxid ausgewählt aus Wasserstoff und/oder einem Alkalimetallsalz davon,
III) Vermischen der carboxylierten Cellulose mit hohem Molekulargewicht aus Schritt I) und dem Peroxid aus Schritt II) und Wasser, schrittweise und in jeglicher Reihenfolge, bei einer Reaktionstemperatur von 50 bis 85°C,
IV) Beibehalten der Temperatur des in Schritt III) erhaltenen Gemischs bis zum vollständigen Verbrauch des Peroxids,
V) Abkühlen des Gemischs auf eine Temperatur unter 50°C, und
VI) gegebenenfalls Neutralisieren der erhaltenen depolymerisierten carboxylierten Cellulose.

**11.** Verfahren nach einem der vorhergehenden Ansprüche, bei dem die depolymerisierte carboxylierte Cellulose ein Carboxymethylderivat und/oder Carboxymethylhydroxypropylderivat und/oder Carboxymethylhydroxyethylderivat von Cellulose ist, bevorzugt ist die depolymerisierte carboxylierte Cellulose depolymerisierte Carboxymethylcellulose.

**12.** Verfahren nach einem der vorhergehenden Ansprüche, bei dem das Verfahren ferner einen Schritt vi) umfasst, bei dem das gefällte Calciumcarbonat von der in Schritt iv) erhaltenen wässrigen Suspension abgetrennt wird, und gegebenenfalls Schritt vii), bei dem das in Schritt vi) erhaltene abgetrennte gefällte Calciumcarbonat getrocknet wird.

**13.** Verfahren nach einem der vorhergehenden Ansprüche, bei dem das Verfahren ferner einen Schritt viii) umfasst, bei dem wenigstens ein Teil der Oberfläche des erhaltenen gefällten Calciumcarbonats mit wenigstens einem Hydrophobierungsmittel nach Schritt iv) und/oder nach Schritt vi), falls vorhanden, und/oder während und/oder nach Schritt vii), falls vorhanden, in Kontakt gebracht wird, bevorzugt ist das wenigstens eine Hydrophobierungsmittel ausgewählt aus der Gruppe bestehend aus einer aliphatischen Carbonsäure mit einer Gesamtmenge an Kohlenstoffatomen von $C_4$ bis $C_{24}$ und/oder Reaktionsprodukten davon, einem monosubstituierten Bernsteinsäureanhydrid, bestehend aus Bernsteinsäureanhydrid monosubstituiert mit einer Gruppe ausgewählt aus einer linearen, verzweigten, aliphatischen und cyclischen Gruppe mit einer Gesamtanzahl an Kohlenstoffatomen von wenigstens $C_2$ bis $C_{30}$ im Substituenten und/oder Reaktionsprodukten davon, einem Phosphorsäureesterblend von einem oder mehreren Phosphorsäuremonoestern und/oder Reaktionsprodukten davon und einem oder mehreren Phosphorsäurediestern und/oder Reaktionsprodukten davon, Polyhydrogensiloxan und Reaktionsprodukten davon, einem inerten Silikonöl, bevorzugt Polydimethylsiloxan, sowie Gemischen davon.

## Revendications

**1.** Processus pour produire une suspension aqueuse de carbonate de calcium précipité comprenant les étapes de :

i) fourniture d'une matière contenant de l'oxyde de calcium,
ii) fourniture d'au moins une cellulose carboxylée dépolymérisée ayant une masse moléculaire $M_w$ dans la plage de 10 000 à 40 000 g/mol,
iii) préparation d'un lait de chaux en mélangeant de l'eau, la matière contenant de l'oxyde de calcium de l'étape i) et l'au moins une cellulose carboxylée dépolymérisée de l'étape ii) pour obtenir un lait de chaux, dans lequel la matière contenant de l'oxyde de calcium et l'eau sont mélangées dans un rapport pondéral de 1:1 à 1:12, et
iv) carbonation du lait de chaux obtenu dans l'étape iii) pour former une suspension aqueuse de carbonate de calcium précipité.

**2.** Processus selon la revendication 1, dans lequel l'étape iii) comprend les étapes de :

a1) mélange de l'au moins une cellulose carboxylée dépolymérisée de l'étape ii) avec de l'eau et a2) ajout de la matière contenant de l'oxyde de calcium de l'étape i) au mélange de l'étape a1), ou

b1) mélange de la matière contenant de l'oxyde de calcium de l'étape i) et
de l'au moins une cellulose carboxylée dépolymérisée de l'étape ii), et
b2) ajout d'eau au mélange de l'étape b1), ou
c) mélange de la matière contenant de l'oxyde de calcium de l'étape i), de l'au moins une cellulose carboxylée dépolymérisée de l'étape ii) et d'eau simultanément.

**3.** Processus selon l'une quelconque des revendications précédentes, dans lequel le processus comprend en outre l'étape v) d'ajout d'au moins un additif d'extinction à l'étape iii) de processus, de préférence l'au moins un additif d'extinction est sélectionné dans le groupe constitué par des acides organiques, des sels d'acide organique, des alcools glucidiques, des monosaccharides, des disaccharides, des polysaccharides, des gluconates, des phosphonates, des lignosulfonates et des mélanges de ceux-ci.

**4.** Processus selon l'une quelconque des revendications précédentes, dans lequel le lait de chaux obtenu dans l'étape iii) a une viscosité Brookfield de 1 à 1 000 mPa·s à 25 °C, plus de préférence entre 5 et 800 mPa·s à 25 °C et le plus de préférence entre 10 et 500 mPa·s à 25 °C, et/ou la suspension de PCC obtenue dans l'étape iv) a une viscosité Brookfield inférieure ou égale à 1 600 mPa·s à 25 °C, plus de préférence inférieure ou égale à 1 500 mPa·s à 25 °C et le plus de préférence inférieure ou égale à 1 400 mPa·s à 25 °C.

**5.** Processus selon l'une quelconque des revendications précédentes, dans lequel la suspension de PCC obtenue dans l'étape iv) a une teneur en solides d'au moins 10 % en poids, de préférence de 15 à 70 % en poids, plus de préférence de 19 à 60 % en poids, même plus de préférence de 21 à 50 % en poids et le plus de préférence de 24 à 42 % en poids, sur la base de la quantité totale de la suspension.

**6.** Processus selon l'une quelconque des revendications précédentes, dans lequel la cellulose carboxylée dépolymérisée a un indice de polydispersité de 2 à 10, de préférence de 2 à 8, plus de préférence de 2,5 à 6 et le plus de préférence de 3 à 5.

**7.** Processus selon l'une quelconque des revendications précédentes, dans lequel la cellulose carboxylée dépolymérisée a le degré de carboxylation de 0,2 à 2, de préférence de 0,4 à 1,8, plus de préférence de 0,5 à 1,6 et le plus de préférence de 0,6 à 1,4.

**8.** Processus selon l'une quelconque des revendications précédentes, dans lequel la cellulose carboxylée dépolymérisée a une masse moléculaire $M_w$ dans la plage de 13 000 à 35 000 g/mol et de préférence dans la plage de 13 000 à 25 000 g/mol.

**9.** Processus selon l'une quelconque des revendications précédentes, dans lequel la cellulose carboxylée dépolymérisée
est prévue en forme d'une solution ayant une teneur en solides de 10 à 60 % en poids sur la base du poids total de la solution, de préférence de 25 à 45 % en poids, plus de préférence de 30 à 40 % en poids et le plus de préférence de 31 à 35 % en poids, et/ou
est ajoutée en une quantité de 0,001 à 5 % en poids sur la base du poids total de la matière contenant de l'oxyde de calcium dans le lait de chaux, de préférence de 0,01 à 2 % en poids, plus de préférence de 0,05 à 1 % en poids, et le plus de préférence de 0,1 à 0,5 % en poids.

**10.** Processus selon l'une quelconque des revendications précédentes, dans lequel la cellulose carboxylée dépolymérisée est préparée en dépolymérisant une cellulose carboxylée de masse moléculaire élevée dans un processus comprenant les étapes suivantes :

I) fourniture d'une cellulose carboxylée de masse moléculaire élevée ayant une masse moléculaire de plus de 40 000 g/mol et un degré de carboxylation dans la plage de 0,2 à 2,
II) fourniture d'un peroxyde sélectionné parmi le peroxyde d'hydrogène et/ou un sel de métal alcalin de ce dernier,
III) mélange de la cellulose carboxylée de masse moléculaire élevée de l'étape I) et du peroxyde de l'étape II) et d'eau de manière incrémentale et dans n'importe quel ordre à une température de réaction de 50 à 85 °C,
IV) maintien de la température du mélange obtenu à l'étape III) jusqu'à consommation complète du peroxyde,
V) refroidissement du mélange jusqu'à une température au-dessous de 50 °C, et
VI) de manière facultative, neutralisation de la cellulose carboxylée dépolymérisée obtenue.

**11.** Processus selon l'une quelconque des revendications précédentes, dans lequel la cellulose carboxylée dépolymérisée est un dérivé de carboxyméthyle et/ou un dérivé d'hydroxypropyle carboxyméthylique et/ou un dérivé d'hydroxyéthyle carboxyméthylique de cellulose, de préférence la cellulose carboxylée dépolymérisée est de la carboxyméthylcellulose dépolymérisée.

**12.** Processus selon l'une quelconque des revendications précédentes, dans lequel le processus comprend en outre une étape vi) de séparation du carbonate de calcium précipité depuis la suspension aqueuse obtenue dans l'étape iv), et de manière facultative une étape vii) de séchage du carbonate de calcium précipité séparé obtenu dans l'étape vi).

**13.** Processus selon l'une quelconque des revendications précédentes, dans lequel le processus comprend en outre une étape viii) de mise en contact d'au moins une partie de la surface du carbonate de calcium précipité obtenu avec au moins un agent hydrophobisant après l'étape iv) et/ou après l'étape vi), si présente, et/ou pendant et/ou après l'étape vii), si présente, de préférence l'au moins un agent hydrophobisant est sélectionné dans le groupe constitué par un acide carboxylique aliphatique ayant une quantité totale d'atomes de carbone de $C_4$ à $C_{24}$ et/ou de produits de réaction de celui-ci, un anhydride succinique mono-substitué consistant en un anhydride succinique mono-substitué par un groupe sélectionné parmi un groupe linéaire, ramifié, aliphatique et cyclique ayant une quantité totale d'atomes de carbone allant d'au moins $C_2$ à $C_{30}$ dans le substituant et/ou de produits de réaction de celui-ci, un mélange d'ester d'acide phosphorique d'un ou plusieurs mono-esters d'acide phosphorique et/ou de produits de réaction de celui-ci et un ou plusieurs diesters d'acide phosphorique et/ou de produits de réaction de celui-ci, de polyhydrogénosiloxane et de produits de réaction de celui-ci, d'une huile de silicone inerte, de préférence de polydiméthylsiloxane et des mélanges de ceux-ci.

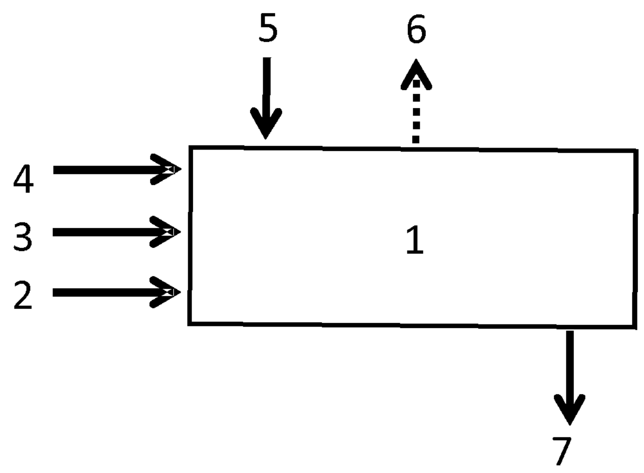


Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011121065 A1
- EP 2537900 A1
- US 2011158890 A1
- EP 0313483 A1
- EP 2447213 A1
- WO 2013142473 A1
- US 6294143 B
- US 5232678 A
- US 5558850 A
- JP 2008074629 A
- EP 0844213 A1
- WO 2010018432 A1
- WO 2005000742 A1
- WO 2004106236 A1
- EP 2929980 A1
- EP 3061729 A1
- KR 100958593 B1
- WO 2007067146 A1
- EP 2524898 A1
- EP 2371766 A1
- DE 1543116 A1
- DE 4411681 A1
- EP 2868716 A1
- EP 2159258 A1
- EP 2390285 A1
- EP 2390280 A1
- WO 2014060286 A1
- WO 2014128087 A1
- EP 2722368 A1
- EP 2770017 A1
- EP 14166751 A


### Non-patent literature cited in the description


- **KATZ et al.** The determination of strong and weak acidic groups in sulfite pulps. *Svensk Paperstidn.,* 1984, vol. 6, 48-53